# EUROPEAN PATENT APPLICATION

(11) **EP 4 792 762 A1**
(43) Date of publication of application: **19.08.2026**
(21) Application number: 24884835.0
(22) Date of filing: 30.10.2024
(51) Int. Cl.: A61B 18/02, A61B 18/12

(54) **FREEZING AND ELECTRIC ABLATION COMBINED ABLATION SYSTEM, ABLATION NEEDLE, AND CONTROL METHOD**

(30) Priority: 30.10.2023 CN 202311419959
(71) Applicant: Medinux (Tianjin) Technologies Co., Ltd., Tianjin 300384 (CN)
(72) Inventor: ZHAO, Guojiang, Tianjin 300384 (CN); HU, Kaiwen, Tianjin 300384 (CN)
(74) Representative: Schüssler, Andrea
(86) International application number: PCT/CN2024/128569
(87) International publication number: WO 2025/092835

(57) **Abstract**

A combined cryoablation and electroablation system, ablation probe, and control method, wherein the ablation system comprises: a control module, freezing and electroablation module connected thereto, with the freezing and electroablation modules connected to the ablation probes. The freezing module freezes the target tissue below 0°C via the ablation probe, performing cryoablation and/or cryofixation and/or cryoanaesthesia on the target tissue. The electroablation module comprises at least two mutually insulated electrodes, with at least one electrode partially situated within a light frozen zone or a deep-frozen zone convertible into light frozen zone, the electroablation module performs electroablation on the target tissue via electrodes, the freezing and electroablation acts in concert on the same target tissue. By selectively controlling the temperature of the frozen zone, effective electroablation is achieved on the periphery of the frozen zone, making the application of electroablation products primarily on the target tissue at the outer edge of the ice ball, enhancing the precision of ablation and improving its efficiency, seamlessly integrating cryoablation and electroablation to overcome their respective shortcomings and allow their strengths to complement one another.

## Description

### CROSS-REFERENCE TO RELATED APPLICATIONS

This application claims the benefit of the Chinese patent application filed with the China National Intellectual Property Administration on 30th October 2023, with application number 202311419959.4, entitled "A combined cryoablation and electroablation, ablation probe, and control method", the entire contents of which are incorporated herein by reference.

### TECHNICAL FIELD

This application relates to the field of medical device technology, particularly to a combined cryoablation and electroablation system, ablation probe, and control method.

### BACKGROUND

Cryoablation or cryosurgery is a therapeutic technique to destroy target tissues by applying low temperatures, widely used for the ablation of tumours, soft tissues, skin, nerves, ducts, and cavities. However, cryoablation technology suffers from several fundamental limitations:
(1) Incomplete ablation persists at the periphery of the frozen zone (0°C to -40°C) and major blood vessels, leading to local recurrence.
(2) Multiple (two or more) freeze-thaw cycles are required, resulting in prolonged operation time and significant resource consumption.
(3) The indiscriminate ablation of cryoablation may cumulatively affect vital organs and healthy tissue.
(4) Extremely low freezing temperatures (below -40°C for reliable inactivation) are needed, necessitating costly cryogens with significant challenges for transportation and storage, such as liquid nitrogen or high-pressure argon gas.
(5) Extremely rapid cooling rates are required (exceeding -25°C/min).

Electroablation is a tissue ablation method that applies electrical energy on target tissues and utilising electric current or field effects for tissue destruction. This includes direct current ablation (electrochemical ablation, electrical pulse/electroporation ablation) and high-frequency alternating current ablation (e.g., radiofrequency ablation, microwave ablation, TTFields tumour alternating electric field ablation). Electroablation is a group of tissue ablation techniques which generated significant attention and experienced rapid advancement in recent years, and widely used in the ablation of skin, soft tissues and organs, blood vessels, cavity tissues, and tumours.

Whilst offering substantial therapeutic benefits, electroablation also presents several critical drawbacks:
(1) Treatment efficacy depends on the electrical properties of the target tissue, resulting in poor selectivity and limited predictability and controllability.
(2) Electroablation does not induce density changes, rendering intraoperative imaging unsuitable for evaluating or monitoring treatment efficacy.
(3) Electrode probes cannot be fixed within the target tissue, leading to probe displacement, unintended injury, or inadequate treatment.
(4) Electrical pulse ablation causes significant patient discomfort, necessitating general anaesthesia and neuromuscular blocking agents, thereby limiting its applicability.
(5) Electrochemical ablation and alternating electric field tumour therapy require treatment durations of several hours, which are difficult for both physicians and patients to tolerate.

Ablation probes or electrodes used for electroablation or combined cryoablation and electroablation are predominantly fabricated from medical-grade stainless steel, such as medical grades stainless steel 304 and 316 SS, which offer advantages of low cost and favourable machinability. However, stainless steel is an iron-chromium alloy with additional elements, containing significant quantities of metals harmful to human health, including: 316 SS contains 16%-18%), nickel Ni (304 SS contains 8%-10.5%, 316 SS contains 10%-14%), manganese Mn (304 and 316 SS contain 2%), and others. During electroablation (e.g., electrochemical or electro-pulse) and combined cryo-electro ablation procedures, stainless steel ablation probes undergo electrochemical corrosion, which leads to substantial uptake of harmful elements into the body, posing significant health risks.

The combined freezing/cryoablation and electroablation technique (also called "cryoelectric ablation" in this document) theoretically leverages the advantages of both cryoablation and electroablation while mitigating their respective limitations. However, integrating cryoablation and electroablation necessitates stringent control over the conditions and methodology of their combination. Failure to do so may result in improper integration under incorrect conditions, thereby completely negating the purpose and efficacy of combined cryoablation and electroablation.

During freezing, the core region where the target tissue contacts the ablation probe typically reaches temperatures below the eutectic point of saline conductivity (-21°C) and becomes non-conductive. This insulates the probe electrodes, preventing electroablation initiation. Concurrently, in peripheral areas of the frozen zone or regions surrounding major vessels, junctions with ice balls, or non-freezable structures (such as bile ducts, trachea, urethra, or cavities), temperatures fail to reach sufficiently low levels (typically below -40°C). This prevents complete tissue destruction, rendering cryoablation ineffective. These areas also thaw prior to the frozen core during natural or active thawing, restoring pre-freezing cellular wall integrity and low conductivity, making the combined freezing and electroablation ineffective, preventing targeted tissue ablation precisely where it is most needed and forfeiting the opportunity for complete target tissue destruction through combined electro-cryoablation.

For electroablation, the inability to stabilise the ablation probe within the target tissue frequently causes displacement, leading to omissions or deviations in the electroablation zone. Cryoablation, however, allows the probe to remain securely fixed within the target tissue, tracking tissue movement without displacement, deviation, or unintended damage to normal tissue. By controlling the freezing temperature, the fixed tissue can be maintained in a conductive state (above -21°C) without impeding the electroablation process.

Conversely, high-voltage electrical pulses (reversible and irreversible electroporation) or high-voltage electrochemical or alternating current therapies employed in electroablation generate immense transient neural electrical stimulation signals (voltages reaching thousands to tens of thousands of volts, currents up to tens of amperes). This induces violent muscle contractions and intolerable pain and trauma for the patient. Such treatments necessitate general anaesthesia and the administration of substantial doses of neuromuscular blocking agents at the affected muscles and nerves. This imposes substantial risks and burdens on surgical conditions (general anaesthesia by anaesthetists required), surgeons, and patients, therefore severely limiting the indications, accessibility, and safety of these therapeutic techniques. Cryotherapy, however, reduces tissue sensitivity to electrical stimulation; temperatures below -21°C can even block neural electrical conduction. Therefore, the controlled and effective combination of freezing and electroablation can enhance the safety and efficacy of electroablation while expanding its indications.

This application addresses the aforementioned technical challenges of combined cryoablation and electroablation by proposing a solution.

### SUMMARY

This application provides a combined cryoablation and electroablation system, ablation probe, and control method, addressing the challenge of synergistically enhancing ablation efficacy through coordinated cryoablation and electroablation.

This application provides a combined cryoablation and electroablation system, wherein said ablation system comprises a freezing module and an electroablation module;
wherein the freezing module and electroablation modules are connected with an ablation probe, which freezes and/or electrically ablates the target tissue;
The freezing module is employed to freeze target tissue below 0°C, forming a frozen zone. This zone is subdivided into a light frozen zone and a deep-frozen zone. The light frozen zone, with a temperature range set between -21°C to 0°C, constitutes a conductive region suitable for electroablation. The deep-frozen zone, with the temperature range set below -21°C, forms a non-conductive region unsuitable for electroablation.

The electroablation module is connected with at least two electrodes, comprising at least one first electrode and at least one second electrode. The first and second electrodes are mutually insulated and connected to the module's two oppositely polarised output ports; at least one of said first electrodes is partially arranged within the light frozen zone or within the deep-frozen zone convertible to the light frozen zone, while said second electrodes are partially arranged within the light frozen zone and/or within human tissue and/or in electrical contact with human tissue;
The freezing and electroablation modules operate in concert to control electroablation to be performed prior to freezing, and/or concurrently with freezing, and/or subsequent to freezing.

In some embodiments, the freezing module may also be a separate freezing device, and/or the electroablation module is a separate electroablation device.

In some embodiments, the ablation system further comprises a control module connected to the freezing and electroablation modules to control their freezing and electroablation operations.

In some embodiments, the freezing module possesses a thawing function, which can selectively freeze or thaw said ablation probe with freezing function connected thereto, to control the electrode-contact area of said probe in a light frozen zone, thereby forming a conductive pathway to enable electroablation, or controlling the electrode-contacting area of said ablation probe to be in a deep-frozen zone to prevent electroablation of said electrode, or controlling the magnitude of the electrical impedance between the electrodes of said ablation probe with freezing function.

In some embodiments, the ablation system further comprises a temperature measurement module connected to either the control module or the freezing module. This module measures target tissue temperature via a separate temperature probe and/or thermocouples positioned on the ablation probe. Temperature feedback is utilised to monitor the extent and temperature of the frozen zone, thereby regulating its electrical conductivity.

In some embodiments, the electroablation module further incorporates an impedance measurement and control module. This module monitors the impedance between electrodes and its variations during cryoablation and/or electroablation. Based on this data, it calculates and regulates electroablation parameters, determines and adjusts electrode positioning and distribution within tissue, identifies and modifies participating electrodes and polarity, configures series/parallel connections and grouping of electrodes, and establishes ablation sequencing.

In some embodiments, the impedance measurement and control module incorporates voltage and current adjustment and distribution circuits. Based on monitored electrode-to-electrode impedance and its variations, it controls the distribution of voltage and current across the main circuit and branch circuits: ensuring average distribution, proportional distribution to impedance magnitude, inverse proportional distribution to impedance magnitude, controls duration and timing of electroablation. It further controls and distributes ablation voltage, current, and ablation energy through each electrode.

In some embodiments, a conductive liquid or medication that is biocompatible, non-harmful to tissue and/or beneficial to ablation is injected into a selected region of the target tissue to increase conductivity and medication concentration within that area; alternatively, a non-conductive or cryoprotective liquid that is biocompatible, non-harmful to tissue and/or beneficial to ablation is injected to reduce conductivity and freezing efficacy within the region.

In some embodiments, the ablation system further comprises an ECG R-wave synchronisation module. This module acquires the patient's electrocardiogram via ECG electrodes and calculates the R-wave period. The control module then regulates the electroablation energy delivered by the ablation module to be applied solely during the refractory period of the R.

In some embodiments, the ablation electrode is positioned within the light frozen zone, with the frozen zone designed to rely on natural thawing of the target tissue rather than actively thawing it. This prioritises electroablation of the light frozen zone. As the target tissue thaws naturally, the temperature within the deep-frozen zone rises to that of the light frozen zone, enabling electroablation of the entire target tissue. This freezing - natural thawing - electroablation process is then repeated.

In some embodiments, the freezing module is controlled to thaw the electrode contact area of the ablation probe, raising its temperature to that of the light frozen zone to establish electrical contact with the target tissue, whilst leaving the deep-frozen zone outside the electrode contact zone unfrozen. This enables electroablation to be performed on the light frozen zone first. Subsequently, as the target tissue thaws naturally, the temperature of the deep-frozen zone rises to that of the light frozen zone, enabling electroablation of the entire target tissue; the freeze-electrode thaw-electroablation process is repeated.

In some embodiments, during the gradual withdrawal of the ablation probe from the target tissue, the frozen zone or electrode zone of the ablation probe is utilised to perform continuous segmental freezing and/or electroablation of the puncture tract.

The present invention further provides an ablation probe for use in the ablation system described in the aforementioned technical scheme. It comprises a probe shaft, at least one electrode, and connecting wires. The electrode is mounted on the probe shaft and connected to the electroablation module via the connecting wires. The non-electrode area of the probe shaft is provided with an electrical insulation layer, with at least part of the electrode located within the light frozen zone.

An ablation probe for use in the ablation system described in the aforementioned technical scheme, comprising a probe shaft, a balloon, at least one electrode, and connecting wires. The electrode is connected to the electroablation module via the connecting wires. The balloon is connected to the end of the probe shaft, with the electrode positioned on the balloon. The non-electrode region of the balloon is provided with an electrical insulation layer, and at least one portion of the electrode is arranged within the light frozen zone.

In some embodiments, when the electrodes of the ablation probe function as anodes or electrodes with a high potential, the electrode material or the outer wall material, cladding, or coating material isolating and protecting the electrode comprises:
materials exhibiting resistance to electrochemical corrosion, including: platinum, platinum group alloys, graphite, graphene, carbon fibre materials, or any combination thereof;
or materials whose electrochemical corrosion products exhibit non-harmful properties, including: combinations of one or more of titanium alloys, magnesium alloys, zinc alloys;
or materials whose electrochemical corrosion products possess beneficial properties for dissolution, including one or more combinations of iron or iron alloy materials.

In some embodiments, the electrical insulation layer comprises a biocompatible polymeric electrical insulation material, wherein the electrical insulation layer is a coating or a film layer.

In some embodiments, the ablation probe is connected to the freezing module via a connecting tube, enabling freezing function in the probe shaft or balloon. The probe shaft further incorporates a first insulation layer, the edge of which protrudes beyond the electrical insulation layer, exposing the cryogenically treated electrode portion within the light frozen zone.

In some embodiments, the differential protrusion range of the first insulation layer edge beyond the electrical insulation layer edge is 1 to 10 millimetres.

In some embodiments, the outer periphery of the distal end of the freezing chamber of the probe shaft is connected to an extension section, with refrigerant unable to reach said extension section. An electrode is provided on said extension section, controlling the electrode in the extension to remain partially within the light frozen zone throughout the freezing process.

In some embodiments, an electrolytic corrosion-resistant barrier section is provided at the junction between the extension section and the probe shaft's freezing chamber, isolating the probe shaft's freezing chamber from the extension section.

In some embodiments, the length of the extension section is 3-15 mm.

In some embodiments, the ablation probe comprises at least two electrodes connected to the electroablation module, mutually insulated and of opposite polarity. One electrode is positioned within the light frozen zone or within the deep-frozen zone capable of conversion to the light frozen zone, while the other electrode is positioned within the frozen zone, within human tissue, or in electrical contact with human tissue. In some embodiments, the device further comprises:
a puncture head connected to the tip of the probe shaft;
a flexible sheath tube, the flexible sheath tube being fitted over the exterior of the probe shaft, the probe shaft being flexible and operatively driving the puncture head to extend or retract within the flexible sheath tube;
a corrugated tube positioned at the inner end of the flexible sheath tube, both ends of said corrugated tube being fixedly connected to the flexible sheath tube via a first fixing ring and a second fixing ring respectively;
a drive wire, which passes through the first fixing ring and is fixedly connected to the second fixing ring, with the drive wire positioned between the corrugated tube and the flexible sheath tube.

In some embodiments, a conduit is provided within the probe shaft, one end of which opens at the electrode of the probe shaft and communicates with the target tissue, while the other end opens externally.

In some embodiments, the external end of the conduit is connected to a suction device, fluid extraction device, vacuum pump, syringe, or infusion pump.

In some embodiments, the ablation probe is a multipolar ablation probe, the probe shaft comprises a frozen zone and is provided with at least two electrodes of opposite polarity, namely a first electrode and a second electrode; the light frozen zone comprises a first light frozen zone located proximally within the frozen zone and a second light frozen zone located distally within the frozen zone; said first electrode is axially positioned within the first light frozen zone; said second electrode being axially positioned at the probe tip within the second light frozen zone;

The first electrode and the second electrode are respectively connected to two oppositely polarised output ports of the electroablation module, with the polarity and electroablation parameters of the first and second electrodes configured by the electroablation module;

An electrical insulation layer is provided in the region between the first and second electrodes and in the non-electrode regions of the probe shaft, with an insulation strength not less than the power supply voltage of the device combined freezing and electroablation and the maximum voltage generated by the electroablation module.

In some embodiments, the probe shaft is a rigid probe shaft or a bendable flexible probe shaft.

In some embodiments, the axial length of the electrode is equal to the axial length of the light frozen zone in which it is located.

In some embodiments, the probe shaft is made of conductive material to form an electrical connection between the first electrode or second electrode and the electroablation module.

In some embodiments, the probe shaft has an exposed portion serving as an electrode.

In some embodiments, a second insulation layer is provided between the tip of the ablation probe and the remaining portion of the probe shaft. Said second insulation layer is formed from vacuum, plastic, or other insulation materials, thereby positioning the second electrode within the light frozen zone.

In some embodiments, the probe shaft is a hollow tube, the probe tip has an outlet for dispensing refrigerant or drugs, and the electroablation module is configured to adjust and configure the polarity of the ablation probe electrodes and ablation parameters based on the polarity of drug molecules, thereby regulating the penetration speed and range of drug molecules between electrodes and within the target tissue.

In some embodiments, the probe shaft incorporates a handle at one end; the ablation probe further comprises an outer probe shaft which is removably fitted over the probe shaft of the ablation probe. This outer probe shaft connects to the handle and is electrically linked to the electroablation module. A first electrode is positioned at the proximal end of the first light frozen zone within the outer probe shaft's frozen zone, while a second electrode is situated at the probe tip within the distal end of the second light frozen zone. The ablation probe functions as an ablation probe with freezing function, electroablation probe, or cryoelectric probe, forming an integral unit with the probe handle for repeated use. The outer probe shaft and ablation probe exhibit excellent thermal and electrical conductivity between them.

In some embodiments, the ablation probe further comprises an outer sheath. The outer sheath is removably fitted over the probe shaft of the ablation probe and is axially slidable along the probe shaft. One end of the outer sheath is connected to the handle of the ablation probe. The outer sleeve comprises a shaft-shaped portion, with a first electrode positioned on said shaft-shaped portion, and a second electrode positioned at the tip of the ablation probe;

The outer sleeve is provided with a slider and a groove, wherein the groove is configured to position the first electrode; The slider is fixedly connected to the shaft-shaped portion and is configured to drive the shaft-shaped portion to slide axially along the probe shaft, thereby driving the first electrode to move correspondingly. This alters the spacing and impedance between the first and second electrodes, ensuring both electrodes are positioned within the light frozen zone.

In some embodiments, the outer sheath constitutes an insulated tube. Sliding the outer sheath adjusts the lengths of the frozen zone and light frozen zone. The first electrode is positioned at the distal end of the insulated tube, ensuring it remains within the light frozen zone of the frozen zone.

The present invention further provides an ablation control method combining freezing and electrocautery, applicable to the ablation system described in any of the aforementioned technical solutions. The ablation control method comprises the following steps:
5100: Insert the ablation probe into the target tissue, with at least two electrodes of opposite polarity positioned within the light frozen zone; or with at least one electrode positioned within the light frozen zone and at least one electrode of opposite polarity positioned within or in electrical contact with human tissue.
S200: Control the freezing module or freezing device to issue freezing commands, freezing the target tissue below 0°C via the freezing-capable ablation probe to form a frozen zone;
S300: Prior to, concurrently with, and/or following freezing, control the electroablation module or electroablation device to issue electroablation commands, performing electroablation via the electroablation electrode first on the light frozen zone and subsequently on the deep-frozen zone.

In some embodiments, the ablation control method further comprises the following steps:
The temperature measurement module measures the temperature of the target tissue to obtain temperature values;
The temperature measurement module transmits the temperature value to the control module, which, upon receiving the temperature value, controls the operational status of the frozen zone and the electroablation module.

In some embodiments, after the formation of the frozen zone is complete, the method further comprises the following steps:
S210: The control module issues a thawing command to the freezing module. Upon receiving the command, the freezing module thaws the frozen zone contacted by the ablation probe with freezing function or cryoelectric probe electrode to the temperature of the light frozen zone surrounding the probe shaft, thereby forming a conductive pathway around the probe shaft electrode;
S220: Following the formation of the conductive channel, electroablation is performed on the periphery of the light frozen zone or frozen zone;
S230: Upon completion of electroablation in the light frozen zone, proceed to ablate the deep-frozen zone.
S110: The control module issues an impedance monitoring command to the impedance measurement and control module. This module monitors and displays the impedance information between electrodes, calculates and controls the ablation parameters for electroablation;
S120: Upon receiving inter-electrode impedance data, the control module plans electrode distribution, which is then implemented by the operator;
S130: Upon receiving the electrode impedance information, the control module plans the electrode grouping, which is then implemented by the operator.

In some embodiments, the ablation control method further comprises the following steps:
S240: Cryo-anaesthetising target tissue (muscle or nerve) using an ablation probe with freezing function, placing it within a frozen zone to render it non-conductive, thereby inhibiting nerve conduction and muscle contraction.

The terms "proximal" and "distal" as used herein shall be interpreted as denoting the end closer to or farther from the user, respectively. For example, when the user holds the handle of the ablation probe, the end of the probe shaft nearer to the handle is the proximal end, and the end of the probe shaft or probe tip farther from the handle is the distal end.

The combined cryoablation and electroablation system, ablation probe, and control method provided herein offer the following beneficial effects:
By controlling the freezing module to freeze the target tissue below 0°C, cryoablation or cryofixation of the target tissue is achieved. When the freezing module serves solely to cryofix the target tissue, the electroablation module can be controlled to perform electroablation. The ablation probe remains fixed within the target tissue, preventing probe migration. The sequence of application of the freezing and electroablation modules is adjusted according to the target tissue characteristics, enabling cryoablation and electroablation to mutually compensate for each other's shortcomings. Cryoablation and electroablation synergistically act upon the same target tissue. The electroablation module comprises at least two mutually insulated electrodes, with at least one electrode portion situated within the light frozen zone. The electroablation module performs electroablation on the target tissue via the electrodes. Electroablation is conducted through the electrodes within the light frozen zone, targeting the periphery of the ice ball (light frozen zone). The electroablation products act upon the target tissue at the outer edge of the ice ball, enhancing ablation efficacy and enabling cryoablation and electroablation to overcome their respective limitations while complementing each other's strengths.

### BRIEF DESCRIPTION OF THE DRAWINGS

To more clearly illustrate the technical solutions of the present invention or the prior art, the drawings required for describing the embodiments will be briefly introduced below. It is evident that the drawings described below merely represent certain embodiments of this invention.
Figure 1 is the first schematic diagram of a combined cryoablation and electroablation system, ablation probe and control method of this invention;
Figure 2 is the second schematic diagram of a combined cryoablation and electroablation system, ablation probe and control method of this invention;
Figure 3 is an effect diagram demonstrating the freezing, natural thawing, and thawing followed by electroablation process by the freezing module of the cryoablation and electroablation combined ablation system of this invention;
Figure 4 is the schematic diagram of freezing, thawing and electroablation by the electroablation probe placed at the periphery of the light frozen zone of this invention;
Figure 5 is the schematic circuit diagram between the cryosurgical and electroablation probe in the combined cryoablation and electroablation ablation system of this invention;
Figure 6 is the first structural schematic diagram of the ablation probe with freezing function with electrodes of this invention;
Figure 7 is a second structural schematic diagram of the ablation probe with freezing function with electrodes of this invention;
Figure 8 is the schematic diagram of the ablation of a cavity tumour by a flexible electroablation probe with cryoablation and electroablation functions of this invention;
Figure 9 is the schematic diagram of the probe placement for tumour ablation at critical structures of this invention;
Figure 10 is a flow diagram illustrating the ablation control method of this invention;
Figure 11 illustrates the structure of a conventional cryoelectric probe and isotherm within the frozen zone of this invention;
Figure 12 illustrates the positional relationship between the electrical insulation layer and the first thermal insulation layer of the cryoablation probe with an extension, along with the isotherm of the frozen zone of this invention;
Figure 13 shows the close-up of the probe shaft extension at point A in Figure 12 of this invention;
Figure 14 illustrates the ablation of perivascular tumours by the cryoelectric probe of this invention;
Figure 15 is the first schematic diagram of the ablation probe with built-in balloon of this invention;
Figure 16 is the second schematic diagram of the ablation probe with built-in balloon of this invention;
Figure 17 is the schematic diagram of the structure of a flexible ablation probe with a flexible sheath tube of this invention;
Figure 18 is the appearance schematic diagram of a multi-electrode ablation probe of this invention;
Figure 19 is the structural schematic diagram of a multi-electrode ablation probe of this invention;
Figure 20 is the structural schematic diagram of a multi-electrode ablation probe with the liquid-injection function of this invention;
Figure 21 is the appearance and structural schematic diagram of a multi-electrode outer sheath ablation probe of this invention;
Figure 22 is the appearance schematic diagram of a variable multi-electrode ablation probe of this invention;
Figure 23 is the schematic diagram of the structure of a variable multi-electrode ablation probe of this invention.

Wherein: 100-Ablation system; 111-Temperature measurement module; 112-Freezing module; 121-Electroablation module; 122-Impedance measurement and control module; 123-Adjustable power supply; 131-Cryoelectric probe; 132-Electrode probe; 140-Temperature probe; 150-Control module; 200-Frozen zone; 210-Light frozen zone (LFZ); 220-Deep-frozen zone (DFZ); 230-Conductive channel; 240-Target tissue; 250- Human body;

10-Probe shaft; 11-Electrode; 12-Electrical insulation layer; 13-First thermal insulation layer; 14-Through tube; 15-Extension section; 16-Balloon; 17-Puncture tip; 18-Flexible sheath tube; 19-Corrugated tube; 20-Probe handle; 21-First electrode; 22-Second electrode; 23-Probe tip; 24-Refrigerant tube; 25-Electrode connection wire; 26-Outer wall of probe shaft; 27-Thermal insulation layer of probe shaft; 28-Second thermal insulation layer; 29-Hollow tube; 30-Connecting tube; 31-Injection port; 32-Discharge port; 33-Refrigerant; 35-Contact spring; 36-Outer probe shaft; 37-Sealant; 38-Slider; 39-Groove; 40-Connector; 42-insulation layer of Inner probe; 43-Outer sliding sleeve; 44-Insulation layer of outer sliding sleeve; 45-Handle spring; 46-Spring plate; 47-Spring plate guide rail; 48-Rod-shaped section; 52-First light frozen zone; 53-Second light frozen zone; 54-Proximal end of frozen zone; 55-Distal end of frozen zone; 56-Distal end of insulation tube.

### DETAILED DESCRIPTION OF THE EMBODIMENTS

The technical solutions of the embodiments of this application shall now be described in detail with reference to the accompanying drawings. It should be understood that the embodiments described herein represent only a portion of the embodiments of this application, not all of them. All other embodiments based on the embodiments of this application without creative work obtained by ordinary technicians skilled in the art fall within the scope of protection of this application.

It should be noted that all directional indications (such as up, down, left, right, front, rear, etc.) in the embodiments of this application are used solely for explaining the relative positions and movements of components in a specific orientation (as shown in the drawings). Should this specific orientation change, the directional indications shall correspondingly alter.

Furthermore, descriptions such as "first", "second", etc., within this application are used solely for descriptive purposes and should not be construed as indicating or implying relative importance or the number of technical features implied. Consequently, features defined as "first", "second", etc., may explicitly or implicitly include at least one such feature. Moreover, technical solutions across different embodiments may be combined, provided such combinations are achievable by a person skilled in the art. Where a combination of technical solutions proves contradictory or unfeasible, such a combination shall be deemed non-existent and falls outside the scope of protection claimed herein.

The embodiments of this application provide a combined cryoablation and electroablation system, ablation probe and control method to address the technical issues in the prior art concerning the combination of cryoablation and electroablation, i.e., the existing methods operate cryoablation and electroablation independently, failing to achieve synergistic effects and result in suboptimal ablation outcomes.

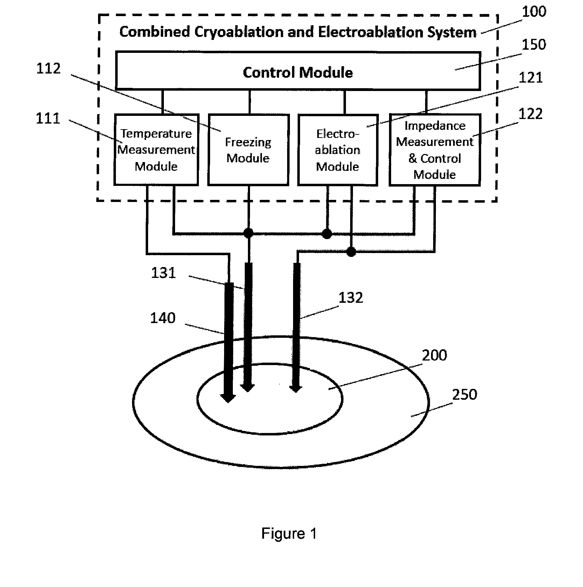

The technical solution in the embodiments of this application addresses the aforementioned technical issues with the following overall approach:
As illustrated in Figures 1 to 3, this application provides a combined cryoablation and electroablation system 100. Said ablation system 100 comprises: a control module 150, a freezing module 112, an electroablation module 121, and a temperature measurement module 111. wherein the freezing module 112, electroablation module 121, and temperature measurement module 111 are all connected to the control module 150; the control module 150, freezing module 112, electroablation module 121, and temperature measurement module 111 are collectively referred to as a cryoelectric ablation system;
the ablation system 100 further comprises an ablation probe, with the freezing module 112 and electroablation module 121 connected to the ablation probe. The ablation probe includes ablation probes with freezing function, electroablation probes, ablation probes with freezing function with electrodes, electroablation probes with freezing capability, and surface electrodes;
an ablation probe with freezing function is an ablation probe possessing solely freezing functionality;
an electroablation probe is an ablation probe solely possessing electroablation functions, namely electrode probe 132;
the ablation probe with freezing function with electrodes is an ablation probe equipped with electrodes and connected to the electroablation module 121, namely the cryoelectric probe 131;
electroablation probes with freezing function are ablation probes connected to a cryogenic agent;
surface electrodes are electrode patches adhered to the skin surface of the human body 250.

The freezing module 112 is employed to freeze the target tissue 240 to below 0°C, forming a frozen zone 200. Through the ablation probe with freezing function, the frozen zone is selectively positioned within the target tissue 240, with controlled refrigerant pressure and flow rate, freezing-power, freezing-duration, and temperature. The resulting ice ball possesses cryoablation functionality and/or cryofixation functionality and/or cryoanaesthesia functionality;
The frozen zone 200 is subdivided into a light frozen zone (LFZ) 210 and a deep-frozen zone (DFZ) 220. The temperature range of the light frozen zone 210 is set between -21°C and 0°C, constituting a conductive zone where electroablation is feasible. The temperature range of the deep-frozen zone 220 is set below -21°C, constituting a non-conductive zone where electroablation is not feasible.

When performing cryoablation with freezing module 112, the target tissue 240 must be frozen to below -40°C;
When performing cryofixation, the freezing module 112 must freeze the ablation probe to below 0°C;
When performing cryoanaesthesia, the freezing module 112 must freeze the ablation probe to below -21°C.

The ablation probe is equipped with electrodes, with the non-active region of the ablation probe insulated. The electrodes comprise at least two electrodes of opposite polarity, mutually insulated from one another.

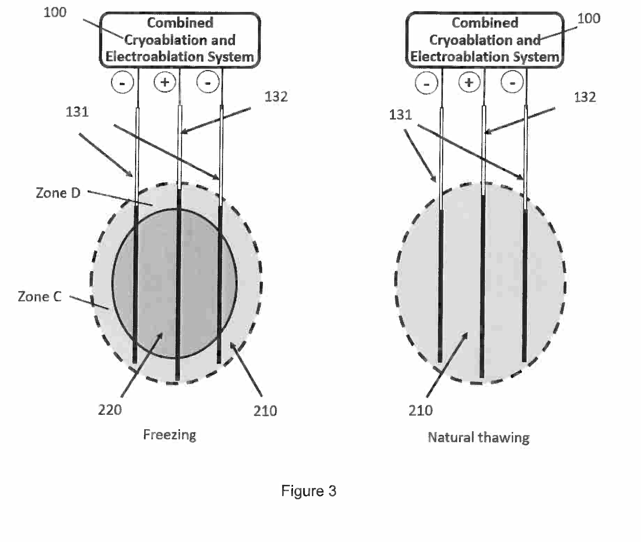

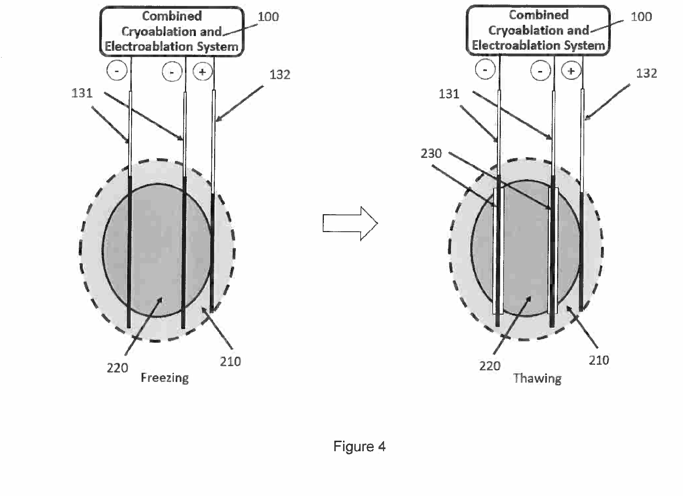

As illustrated in Figures 3 and 4, the electroablation module 121 performs electroablation on the target tissue 240 via electrodes. Said electroablation module 121 is connected to at least two electrodes, comprising at least one first electrode and at least one second electrode. The first electrode and second electrode are mutually insulated and connected to two output terminals of the electroablation module 121 with opposite polarities; at least one portion of said first electrode is located within the light frozen zone 210 or within the deep-frozen zone 220 that can be converted into the light frozen zone 210; said first electrode and second electrode are mutually insulated and have opposite polarities, with at least one portion of said second electrode arranged within the light frozen zone 210, or the human tissue, or in electrical contact with human tissue. Preferably, at least one of said first electrode portions is situated within the light frozen zone 210 after the ice ball is fully formed, enabling electroablation of the target tissue 240 even after the ice ball is fully formed. Although the ice ball inevitably contains both a deep-frozen zone 220 and a light frozen zone 210, and the ice ball naturally thaws, the electrolysis will also occur within the light frozen zone 210. However, positioning the electrodes within the light frozen zone 210 at the ice ball's maximum shape is unprecedented. Upon complete formation, the ice ball attains its predetermined maximum shape, maximising the electroablation range. This allows the electroablation products to exert maximum effect on the outer periphery of the target tissue 240, achieving prior ablation of the tumour periphery for more thorough ablation. At least two mutually insulated electrode segments with opposite polarities are positioned within the light frozen zone 210; alternatively, at least one first electrode segment resides within the light frozen zone 210, while at least one second electrode is externally positioned as a surface electrode or internally implanted.

Specifically, multiple ablation probes with freezing function are positioned in the outer periphery of the electroablation probe, forming a larger frozen zone 200 with an expanded deep-frozen zone 220, suitable for larger tumours. Within the light frozen zone 210, electrodes on the ablation probes with freezing function conductively connect with electrodes on the electroablation probe, enabling electroablation within this zone.

Multiple electroablation probes are positioned in the outer ring of the ablation probes with freezing function. Alternatively, multiple ablation probes with freezing function may be configured, with multiple electroablation probes arranged in the outer ring of these ablation probes with freezing function. The deep-frozen zone 220 can be adjusted by varying the number of ablation probes with freezing function according to tumour size, accommodating tumours of differing dimensions. The ablation probes with freezing function freeze the target tissue 240. Electroablation probes are arranged within the light frozen zone 210, performing electroablation within this zone. Electrical conductivity exists between the anode of the ablation probe with freezing function and the cathode of the electroablation probe, facilitating electrochemical reactions. Electrochemical byproducts from multiple electroablation probes can be extensively distributed within the light frozen zone 210. This effect is particularly pronounced with electrodes incorporating iron, magnesium, or a combination thereof. These byproducts induce toxicity in tumour cells within the light frozen zone 210, thereby enhancing the thoroughness of electroablation within this zone.

A combined cryoablation and electroablation system 100, wherein said ablation system 100 comprises a freezing module 112 and an electroablation module 121;
wherein the freezing module 112 and electroablation module 121 are connected to an ablation probe, which freezes and/or electro-ablates target tissue 240;
the electroablation module 121 comprises at least two mutually insulated electrodes, with at least one electrode positioned on the ablation probe; the electroablation module 121 performs electroablation on target tissue 240 via said electrodes;
electroablation is performed prior to freezing. The electrochemical products of the electrodes possess properties that induce ferrocyanide-mediated cell death, specifically comprising iron, iron alloys, or iron-magnesium alloys.

The ablation probe is inserted into the tumour, where electroablation occurs prior to freezing. During electrochemical ablation, a sacrificial anode allows iron ions to diffuse into the tissue and cells, killing cancer cells. Electrolytic products include iron ions and magnesium ions. Upon entering cells through electroporated membranes, iron ions initiate lipid peroxidation via the Fenton reaction. Beyond acidic and alkaline byproducts, these electrochemical products inflict further damage on tumour cells, leading to their demise. Crucially, iron and magnesium ions are essential elements for the human body, ultimately being absorbed without adverse effects.

The freezing module 112 and electroablation module 121 may constitute an integrated device connected to a common control module 150, as illustrated in Figure 1.

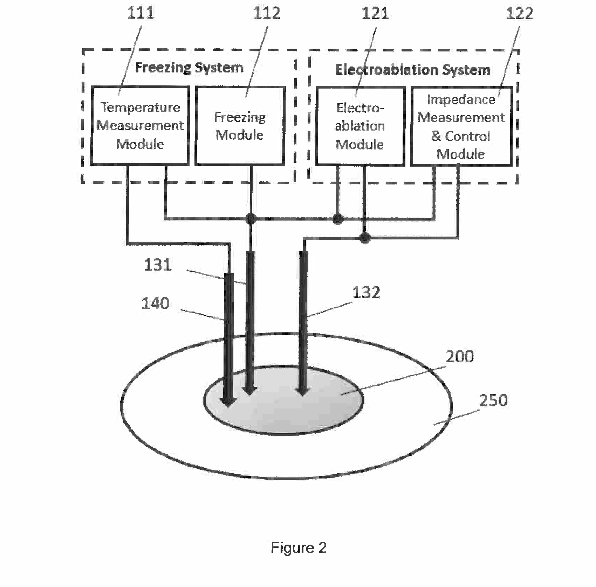

Alternatively, freezing module 112 and electroablation module 121 may comprise separate freezing and electroablation devices, which combine for use to provide both freezing and electroablation capabilities, as illustrated in Figure 2, wherein the freezing device connects to an ablation probe with freezing function and the electroablation device connects to an electroablation probe. The electroablation device may also connect to the ablation probe with freezing function of the freezing device. Furthermore, the separate electroablation device may be configured such that at least one of said electrode portions is positioned within the ice ball region below 0°C. Such combined device configurations also fall within the scope of protection of the present invention's embodiments.

Specifically, the separate freezing device and separate electroablation device may comprise:
a standalone freezing device, wherein an external power source is connected to the ablation probe with freezing function to enable electrochemical ablation functionality;
a separate radiofrequency ablation and pulsed ablation device, wherein the radiofrequency ablation and pulsed ablation device utilises current on the ablation probe to induce electrochemical reactions within the target tissue 240 via the ablation probe on the radiofrequency ablation and pulsed ablation device, thereby achieving the electrochemical ablation function of the radiofrequency ablation and pulsed ablation device.

Following ablation completion, during the gradual withdrawal of the ablation probe from target tissue 240, the frozen zone or electrode zone of the ablation probe is employed to perform continuous segmental freezing and/or electroablation along the puncture tract.

During the actual probe withdrawal process, the ablation probe is energised at regular intervals to induce electrochemical reactions. With each segmental withdrawal, electrochemical products remain within the probe-punctured tissue cavity 250 until the entire cavity undergoes electrochemical ablation. Tumour cells carried out by the ablation probe are destroyed by these electrochemical products, preventing their survival within the human tissue cavity 250. And/or, with each segmental withdrawal, the freezing module 112 performs freezing along the probe track.

A separate freezing device freezes the target tissue 240, providing a conductive environment for electrodes within the frozen zone 200. This freezes the target tissue 240 to form a deep-frozen zone 220 and a light frozen zone 210, enhancing the conductivity of electrodes within the light frozen zone 210. A separate electroablation device controls the electroablation process, performing electroablation on the light frozen zone 210. Tumour cells within the deep-frozen zone 220 are ablated via cryoablation.

The ablation system 100 further comprises a temperature measurement module 111, which connects to either the control module 150 or the freezing module 112, wherein said temperature measurement module 111 measures the temperature of the target tissue 240 via a separate temperature measurement probe 140 and/or a temperature measurement thermocouple pair located on the ablation probe. Temperature feedback is used to monitor the extent and temperature of the frozen zone 200, thereby assisting in controlling the conductivity of the frozen zone 200. Specifically, the temperature measurement module 111 detects the temperature of the frozen zone 200. By feeding back temperature information to the control module 150, the control module 150 regulates the temperature of the frozen zone 200 to remain between -21°C and 0°C, thereby maintaining the conductivity of the frozen zone 200;

The freezing module 112 and the electroablation module 121 act synergistically upon the same target tissue 240. The electroablation is performed prior to freezing treatment, and/or concurrently with freezing treatment, and/or subsequent to freezing treatment.

The insulated region of the ablation probe is positioned within the light frozen zone 210, enabling electrical conduction through the electrode on the probe within this zone. The temperature gradually increases from the centre to the periphery of the ice ball, ensuring the electrode coverage area always includes portions within the light frozen zone 210. During electroablation, this enables conduction between the two electrodes. The control module 150 regulates the freezing range and temperature based on measurements from the surgical temperature measurement module 111, thereby maintaining the temperature within the target tissue 240 at a constant level within the light frozen zone 210. This stabilises the scope of electroablation.

Control module 150 manages the synchronisation between electroablation module 121 and temperature measurement module 111 during probe ablation, ensuring they operate sequentially without interference. Specifically, the control module 150 disconnects the temperature measurement module 111 prior to electroablation by module 121. Given that the rated current of the temperature sensor's detection circuit is approximately 35mA, and taking the voltage applied by ablation module 121 to the human body 250 as 30V as a baseline, with the impedance of target tissue 240 in the human body 250 being approximately 300Ω, resulting in an electroablation current within the human body 250 of approximately 100mA. This significantly exceeds the rated current of the thermocouple detection circuit. Therefore, during electroablation, either the output function of the electroablation module 121 or the temperature measurement module 111 must be disabled, and the thermocouple disconnected to prevent overload damage to the corresponding detection circuit.

By controlling freezing module 112 to freeze target tissue 240 below 0°C, cryoablation or cryofixation of target tissue 240 is performed. When freezing module 112 solely serves to cryofix target tissue 240, electroablation module 121 may be activated to perform electroablation. The ablation probe is fixed within the target tissue 240 to prevent probe migration. The sequence of application for freezing module 112 and electroablation module 121 is adjusted according to the target tissue 240's condition, enabling cryoablation and electroablation to mutually compensate for each other's shortcomings. Cryoablation and electroablation synergistically act upon the same target tissue 240. The electroablation module 121 comprises at least two mutually insulated electrodes, with both electrodes partially situated within the light frozen zone 210. Electroablation is performed via electrodes within the light frozen zone 210. The ablative products act upon the target tissue 240 at the periphery of the ice ball, enhancing ablation targeting and efficiency. This enables freezing and electroablation to overcome their respective limitations and complement each other's strengths.

In conventional freezing procedures, electrical conduction between electrodes at the ice ball's centre and periphery only occurs as the ice ball melts. Consequently, post-melting electroablation is confined to the melted ice ball's perimeter, resulting in suboptimal ablation efficacy for peripheral target tissue 240.

The initial freezing module 112 freezes the target tissue 240. Electrodes within the deep-frozen zone 220 remain non-conductive, while those in the light frozen zone 210 become conductive. Electroablation is performed via electrodes in the light frozen zone 210, targeting the periphery of the ice sphere (light frozen zone 210). The electroablation products act upon the peripheral target tissue 240 outside the frozen zone 200, enhancing ablation efficacy at the periphery of target tissue 240.

Temperature detection modules monitor temperatures within the target tissue 240. The controller then adjusts the freezing intensity applied by the freezing module 112 based on these readings, ensuring more stable and synergistic coordination between the freezing module 112 and the electroablation module 121. By freezing the target area, it is maintained in a low-temperature cryogenic state and becomes cryogenically marked. This enables CT and other density imaging equipment to more clearly delineate the precise ablation boundaries of the target area, thereby defining the ablation zone for subsequent electroablation procedures.

### Embodiment 1: Cryoablation Combined with Electrochemical Ablation

In this embodiment, ablation is performed by combining cryoablation with electrochemical ablation.

During cryoablation combined with electrochemical ablation, both cryosurgical and electroablation probes are inserted into the same target tissue 240. The ablation probes with freezing function may also incorporate electrodes, enabling synergistic action between cryoablation and electrochemical ablation throughout the ablation process.

As illustrated in Figures 1 to 5, the ablation system 100 further comprises an impedance measurement and control module 122, which incorporates voltage and current adjustment and distribution circuits. Based on monitored impedance values and their variations between electrodes, this module controls the distribution of voltage and current across the main circuit and branch circuits. It manages distribution methods including: equal distribution, proportional or inverse proportional distribution to impedance magnitude, control of electroablation duration, and time-division power allocation. It regulates and distributes ablation voltage, current, and ablation energy through each electrode. The impedance measurement and control module 122 connects to the control module 150 and the ablation probe to monitor impedance and its variations between electrodes during cryoablation and/or electroablation. Based on this data, it calculates and controls ablation parameters, i.e., waveform, voltage, current, and duration, to determine and adjust electrode positioning and distribution within tissue, select and modify participating electrodes and polarity, configure series/parallel connections and grouping, and establish ablation sequence. This enables real-time adjustment of electrode distribution based on impedance analysis. During probe insertion, the system periodically detects short circuits or open circuits between electrodes within the target tissue 240, providing immediate status awareness. Should such faults occur, the physician may immediately replace or reposition the electrodes to prevent short circuits or open circuits from affecting the radiofrequency ablation once ice balls have formed.

Furthermore, the freezing module 112 incorporates a thawing function, enabling selective freezing or thawing of connected ablation probes with freezing function. This controls the electrode contact zone of the ablation probe to reside within the light frozen zone 210, thereby forming a conductive pathway 230 to achieve electrocautery ablation. Alternatively, it may control the contact zone of the ablation probe's electrode to reside within a deep-frozen zone 220 to inhibit electroablation at that electrode, or regulate the electrical impedance of the ablation probe's electrode contact zone.

Electrode-equipped ablation probes with freezing function and electroablation probes are inserted into the same target tissue 240. This embodiment employs three electrode-equipped ablation probes with freezing function and one electroablation probe. The three ablation probes CE1, CE2, CE3 are electrode-equipped ablation probes with freezing function (cryoelectric probes 131), functioning as cathodes and arranged in parallel; The electroablation probe AN is an electroablation probe, acting as the anode and connected to the other output terminal of the power supply. The cryoelectric probes 131 are connected in series with the electroablation probe across the two terminals of the power supply.

Total electroablation probe ablation current: $I = {I}_{a 1} + {I}_{a 2} + … + {I}_{an}$

Uncontrolled electroablation currents: The current in each electrode branch is inversely proportional to its impedance ${I}_{a 1} : {I}_{a 2} : … : {I}_{an} = 1 / {Z}_{a 1} : 1 / {Z}_{a 2} : … : 1 / {Z}_{an}$

Through adjustable power supplies 123 and an adjustable shunt circuit (the adjustable shunt circuit constitutes an existing circuit application and is not a subject matter of the present invention; hence, its specific circuit diagram is not reproduced herein), the adjustable shunt circuit can control the current flowing through the ablation probe with freezing function and electroablation probe with electrodes, achieving the following current control effects:
(1) Equal current distribution: Current is equally distributed among each electrode branch, i.e. ${I}_{\left(a1\right)} = {I}_{\left(a2\right)} = … = {I}_{\left(an\right)} = I / n$
(2) Impedance-proportional current distribution: The lower the impedance of each electrode branch, the smaller the current, which is suitable for actual ablation conditions, i.e. ${I}_{a 1} : {I}_{a 2} : … : {I}_{an} = {Z}_{a 1} : {Z}_{a 2} : … : {Z}_{an}$
(3) Sequential electroablation: electroablation is performed in groups using the cryoelectric probe 131 and electroablation probe, proceeding sequentially in time. For example: CE1^{~}AN; CE2^{~}AN; CE3^{~}AN, etc., where ${I}_{\left(a1\right)} = {I}_{\left(a2\right)} = … = {I}_{\left(an\right)} = I$

During the procedure, the centre and edge coordinates of the target tissue 240 are first determined using medical imaging equipment such as CT (Computed Tomography), ultrasound, or MRI scanning. The surgeon configures electrode distribution based on the size and shape of target tissue 240. Upon insertion of the electrode-equipped ablation probe with freezing function and electroablation probe into target tissue 240, control module 150 assesses whether the impedance between ablation probes meets surgical requirements via impedance data measured by impedance monitoring module 122. Should impedance prove excessively high or low, this may indicate an open circuit or short circuit between electrodes. The control module 150 plans the electrode distribution based on the impedance data. Simultaneously, imaging equipment rescans the ablation probe with freezing function and electroablation probe equipped with electrodes. The final configuration is displayed on the monitor. Alternatively, the control module 150 may adjust the polarity of electrodes on the ablation probes and the grouping of multiple electrodes to better align the equipped ablation probes with freezing function and electroablation probes with the preset requirements.

### Embodiment 2: Cryoablation Combined with Electrical Pulse Ablation

The freezing module 112 performs cryoablation on the target tissue 240. As cryoablation is less effective at the periphery of the target tissue 240, the electroablation module 121 supplements ablation at the periphery of target tissue 240. Furthermore, during the period when the electroablation probe conducts high-voltage electricity, electrochemical reactions occur at the probe tip, generating acidic or alkaline solutions that also contribute to the ablation of target tissue 240. The combination of cryoablation and electroablation further enhances ablation efficacy on target tissue 240. Furthermore, the ablation probe incorporates insulation features, with electrodes on the probe fabricated from platinum, platinum group alloys, graphite, graphene, or carbon fibre materials, either individually or in combination. This prevents electrolysis at the electrodes, thereby avoiding electrochemical displacement of the ablation probe itself. This approach prevents probe fracture within the body and maintains its pressure resistance.

During electrical pulse ablation, the frozen zone 112 first freezes the target tissue 240 to form a frozen zone 200. The electrodes within the frozen zone 200 are arranged in groups, with a potential difference existing between electrodes within each group. Electrical pulse ablation occurs within the frozen zone 200, with the electrodes at least partially located within the light frozen zone 210, thereby achieving electrical pulse ablation of the light frozen zone 210.

### Embodiment 3: Radiofrequency Ablation, Microwave Ablation and Electric Field Ablation (TTF)

The electroablation probe is inserted into the target tissue 240, whereupon electrical current is applied. The radiofrequency electrode emits radiofrequency waves or microwaves, which disrupt tumour cells within the target tissue 240. This interference impairs the normal growth of tumour cells, causing disorganisation in their growth and preventing normal division and proliferation, thereby completely deactivating the zone.

In this embodiment, ablation is performed by combining cryoablation with either radiofrequency, microwave, or electric field ablation. The freezing module 112 performs cryoablation on the target tissue 240. As cryoablation is less effective at the periphery of the target tissue 240, the ice ball freezes the target tissue 240. Upon freezing, water within the tissue crystallises, precipitating salts and forming conductive salt channels within the target tissue 240. This enables conductivity within the light frozen zone 210. The electroablation module 121 supplements ablation within the light frozen zone 210. The combination of cryoablation with radiofrequency ablation, and microwave ablation with electric field ablation (TTF), further enhances ablation efficacy within the target tissue 240.

Preferably, the selection and timing of electroablation versus cryoablation are categorised as follows:
Cryoelectric ablation: employing the synergistic effects of electroablation and cryoablation on target tissue 240 to achieve composite, superimposed ablation. This specifically involves the following application sequence:
1) Upon completion of the electroablation module 121, the frozen zone 112 is immediately activated for cryoablation: the electroablation probe acts on major vessels near the target tissue 240, then the electroablation module 121 is controlled to reduce blood flow velocity within these vessels, thereby diminishing heat generated by blood circulation. Subsequently, the frozen zone 112 is rapidly initiated, accelerating its cooling rate to swiftly form an ice ball. The target tissue 240 is rapidly cooled to the required cryoablation temperature. Concurrent application of both electroablation and cryoablation to the target tissue 240 enhances ablation efficacy.
   Naturally, electroablation may follow cryoablation immediately. Cryoablation permeabilises tumour cell walls, allowing electroablation to directly target nuclei deprived of cellular wall protection, thereby further enhancing ablation efficacy.
2) The electroablation module 121 and frozen zone 112 activate simultaneously. During the cooling process of the frozen zone 112 on the target tissue 240, cooling originates from the ablation probe. The core temperature of the formed ice ball is lowest, gradually reaching 0°C towards the periphery. The ice sphere exhibits electrical conductivity only within the temperature range of -21°C to 0°C. While the freezing module 112 cools the target tissue 240, the electroablation module 121 performs electroablation on the target tissue 240. As the ice sphere formed by the freezing module 112 gradually expands, the temperature of the target tissue 240 progressively decreases. With the lowering temperature of the ice sphere, the extent of the deep-frozen zone 220 within the frozen zone 200 progressively expands until it encompasses the electrode. At this point, the electroablation module 121 ceases operation due to an open circuit formed by the electrode. During the period when the ice sphere gradually grows to its maximum size and the deep-frozen zone 220 progressively covers the electrode, the electroablation module 121 performs electroablation on the target tissue 240. Electrocoagulation products will diffuse outward from the centre of the ice ball as it enlarges. As part of the electrode remains exposed within the light frozen zone 210, the electrocoagulation module 121 will simultaneously continue electrocoagulating target tissue 240 within the light frozen zone 210 during cryotherapy. The electroablation and cryoablation from module 121 simultaneously ablate target tissue 240, allowing the ablation effects of cryoablation and electroablation to be combined, thereby enhancing ablation efficacy.
   Particularly when using electrochemical ablation, the electrochemical by-products persist within the ice ball even after the electroablation module 121 is deactivated. The synergistic action of cryoablation and electrochemical ablation on the target tissue 240 yields superior ablation outcomes.
3) Following freezing by the freezing module 112, the electroablation is performed immediately by electroablation module 121. After the freezing module 112 concludes its operation, the inner zone of the target tissue 240 is ablated and the tumour cells on the outer periphery of the target tissue 240 is permeabilised. Electroablation is then applied, enhancing the electroablation efficacy against tumour cells within the target tissue 240.

The conventional thawing operation of freezing module 112 serves as preparatory work for subsequent cryoablation. The traditional cryoablation procedure involves first freezing target tissue 240 to form an ice ball. At this stage, the ice ball has not yet reached the required size. Ice possesses a certain degree of thermal insulation, preventing the low temperature at the ablation probe from absorbing external heat. Consequently, a single freezing cycle cannot produce a larger ice ball. Thawing causes the ice ball to fracture internally, forming a dense network of divergent cracks. Upon re-freezing, external temperatures can then conduct into the interior, thereby enlarging the ice ball. Another purpose of the thawing operation in the conventional freezing module is to facilitate probe extraction. As the ice ball becomes frozen to the ablation probe, it may take tens or even several tens of minutes to thaw completely after cryoablation. To enable rapid probe withdrawal, the probe must be warmed to thaw the ice around it at the probe site, allowing swift removal.

Alternatively, at least two mutually insulated electrodes may be provided on the same ablation probe, with both electrodes situated within the light frozen zones 210 at either end of the frozen zone 200. Following freezing of the target tissue 240 within the frozen zone 200 formed by the frozen zone 112, the two electrodes on the same ablation probe are positioned within the light frozen zones 210. The electroablation module 121 then performs electroablation via the electrodes within the light frozen zones 210. This enables simultaneous electroablation and cryoablation, generating greater electrochemical products within the same timeframe. Furthermore, as the two electrodes are positioned at opposite ends of the light frozen zones 210, enabling electroablation to cover the entire light frozen zone 210 for more thorough ablation. Particularly when ablating small tumours, insertion of a single ablation probe suffices, ensuring the frozen zone 200 encompasses the tumour and reducing probe insertion complexity during surgery.

Preferably, as illustrated in Figure 3, the frozen zone 112 of the present invention incorporates a thawing function. The control module 150, receiving temperature feedback from the temperature measurement module 111, can selectively control the frozen zone 112 to freeze and/or thaw ablation probes with cryoablation function. This transforms the frozen zone 200 contacting the electrodes on the ablation probe into a light frozen zone 210, thereby enabling electrical conduction between the electrodes on the ablation probe and the periphery of the target tissue 240 to achieve the conductive properties required for electroablation. This forms a conductive pathway 230 to perform electroablation on the selected frozen zone 200. This embodiment illustrates an ablation method employing one electroablation probe surrounded by two or three ablation probes with freezing function equipped with electrodes. Re-warming of the freezing module 112 partially thaws the electrode section to above -21°C. This aims to maintain the frozen zone 200 in contact with the ablation probe's electrode at temperatures above -21°C, transforming it into a light frozen zone 210. Furthermore, the electroablation probe penetrates the periphery of the ice ball, creating a temperature zone above -21°C between the electrodes in the deep-frozen zone 220 and the light frozen zone 210. As ice becomes electrically conductive above -21°C, the electrodes on the cryoelectric probe 131 can conduct electricity with the outer periphery of the ice ball. This forms a conductive pathway 230 between the cryoelectric probe131 along the ice ball's longitudinal axis and the ice ball's outer periphery. Consequently, the electroablation module 121 performs electroablation on the ice ball's outer periphery via the electrodes and conductive pathway 230. The external impedance of the ice ball far exceeds that at the conductive channel 230, thereby delimiting the electroablation zone. The ice ball immobilises the tumour, preventing its escape from the electroablation field. As incomplete ablation may occur within the ice ball during cryoablation, the temperature at the electrode on the cryoelectric probe 131 gradually rises above -21°C as thawing progresses. The ablation probe with freezing function with its electrode and the electroablation probe then forms a conductive pathway 230 between the ice ball's minor axes, enabling electroablation of the target tissue 240 internally. Crucially, ablation prioritizes the tumour periphery via the conductive pathway 230 before synergistically ablating the tumour interior through combined cryoablation and electroablation, achieving more thorough tumour destruction.

Electroablation is performed concurrently with and/or following cryoablation. The electrode is positioned such that part of it lies within the selected light frozen zone 210 while maintaining electrical contact with the target tissue 240, controlling the freezing module 112 to refrain from actively thawing the target tissue 240 during or after cryotherapy, relying instead on natural thawing. This allows electroablation to prioritize ablation of the selected light frozen zone 210, such as zones at the periphery of the ice ball, at ice ball junctions, and around major vessels. Subsequently, as natural thawing progresses, the temperature of the deep-frozen zone 220 rises to that of the light frozen zone 210, enabling electroablation of the entire target tissue 240. This cryoablation - natural thaw - electroablation process is then repeated.

Electroablation is performed concurrently with cryoablation and/or following cryoablation. The freezing module 112 or freezing device partially thaws the contact area of the electrode on the ablation probe with freezing function, thereby thawing the electrode. This raises the temperature of the electrode contact zone above -21°C without thawing the deep-frozen zone 220 outside the electrode contact area of the ablation probe with freezing function. This establishes electrical conduction between the electrode and the target tissue 240, thereby forming a conductive pathway 230 to first perform electroablation on the light frozen zone 210. Subsequently, as active thawing progresses, the temperature of the deep-frozen zone 220 rises to that of the light frozen zone 210, enabling electroablation of the entire target tissue 240. This cryoablation-electrode thaw-electroablation process is repeated.

By operating at least one cycle of the aforementioned cryoablation-thaw-electroablation sequence, the extent of the light frozen zone 210 is maintained relatively constant. Consequently, the zone affected by electroablation products is concentrated within the light frozen zone 210, prioritizing ablation of the outer periphery of the target tissue 240. This approach prevents incomplete ablation of the tumour's outer periphery.

Pure electroablation: The freezing module 112 cools the target tissue 240 to a non-freezing ablation temperature below 0°C, specifically between -21°C and 0°C. The ice crystals formed within the frozen target tissue 240 serve solely to anchor the ablation probe. Electroablation is then performed, preventing probe displacement during the procedure and enhancing probe fixation. Furthermore, the ablation zone is confined to the frozen target tissue 240, ensuring more precise ablation targeting and improved ablation efficacy. Concurrently, within the frozen target tissue 240, tumour cells undergo dehydration, causing salts to precipitate alongside water. This forms conductive salt channels within the target tissue 240. These conductive channels reduce the impedance of the electrical circuit within the target tissue 240, thereby enhancing the efficacy of the electroablation.

Furthermore, during the operation of the freezing module 112 and electroablation module 121, the electrode becomes at least partially frozen within the target tissue 240 or at its periphery, wherein the freezing module 112 controls the electrode portion to remain within the light frozen zone 210 via temperature feedback from the temperature measurement module 111. Specifically, the temperature measurement module 111 transmits measured temperature data to the control module 150, which then directs the freezing module 112 to freeze or thaw based on this data, ensuring the electrode portion remains within the light frozen zone 210.

Ablation probes comprise ablation probes with freezing function, electroablation probes, ablation probes with freezing function with electrodes, and electroablation probes with freezing functionality.

Specifically, when the ablation probe is an ablation probe with freezing function, an electroablation probe (electrode probe 132), or an ablation probe with freezing function with electrodes (cryoelectric probe 131), the ablation probe is first inserted into the target tissue 240. The freezing module 112 then cools the target tissue 240, causing the ablation probe to freeze into the target tissue 240.

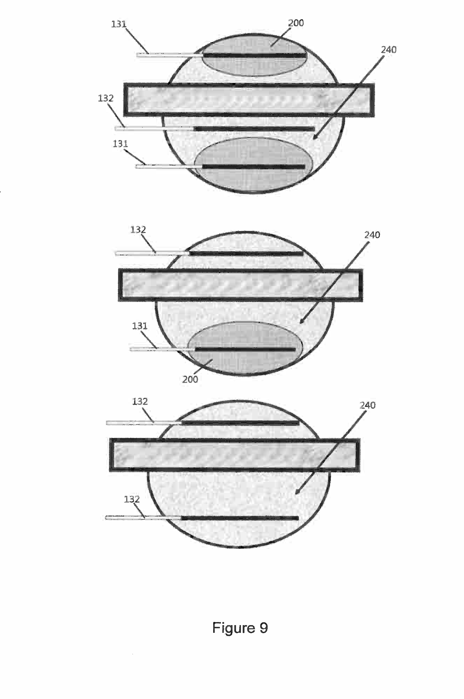

Furthermore, as illustrated in Figure 9, when tumours are situated near blood vessels, cavities, or critical organ structures, probe placement becomes constrained. For instance, tumours adjacent to major vessels cannot be frozen into ice balls due to the continuous heat supply from blood flow. Consequently, ablation probes with freezing function cannot fully encapsulate the tumour within an ice ball. To address this cryoelectric probes 131 are deployed along the tumour margins on both sides of the vessel, with electroablation probes positioned between these ablation probes with freezing function and the vessel itself. The deep-frozen zone 220 of the cryoelectric probe 131 spans the tumour margins on both sides of the vessel. The perivascular zone can only form a light frozen zone 210 or remain unfrozen. Electroablation is performed via the cryoelectric probe 131 and electroablation probe, with electrochemically generated products transferred between these probes and diffusing within the tumour. This achieves ablation of tumours situated near blood vessels, body cavities, or vital organ structures.

Alternatively, for tumours adjacent to vital organs where only the tumour margin on one side of the organ permits cryogenic ice ball formation, as illustrated in Figure 9 for this embodiment, where insufficient insertion space exists for multiple ablation probes within the target tissue 240, an cryoelectric probe 131 is positioned at the tumour margin adjacent to the vital organ. Electroablation probes are deployed at the tumour margin on the opposite side where a cryoelectric probe 131 cannot be placed, alongside an electrode probe 132. The freezing module 112 then cools the cryoelectric probe 131, creating a deep-frozen zone 220 at the tumour margin adjacent to the vital organ. This establishes relative fixation between the ablation probe and the tumour. A single electrified ablation probe with freezing function simultaneously performs local cryoablation and cryofixation, facilitating easier insertion of the electrode probe 132. Electroablation is then conducted by both the cryoelectric probe 131 and the electrode probe 132. Electrochemical products are transferred between the cryoelectric probe 131 and the electrode probe 132, diffusing within the tumour to achieve ablation of tumours located near blood vessels, cavities, or vital organ structures;

Alternatively, for tumours adjacent to vital organs, as illustrated in Figure 9, where insufficient space exists for multiple ablation probes to penetrate the target tissue 240 concurrently, nor adequate freezing zone is available, only the ablation probe with freezing function with electrodes is inserted. Electroablation is then performed using two or more electrode probes 132, or a surface electrode plate combined with at least one electrode probe 132.

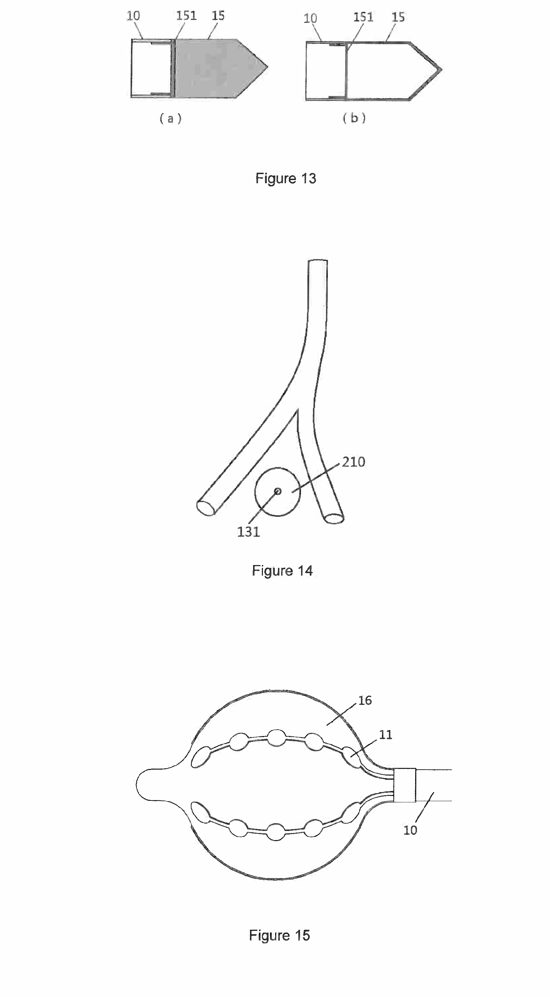

Alternatively, for tumours situated at vascular intersections, as illustrated in Figure 14 for this embodiment, the persistent heat influx from vascular flow prevents the formation of a deep-frozen zone 220. Instead, an ablation probe with freezing function is inserted to create a light frozen zone 210 at the vascular intersection. Subsequently, the cryoelectric probe is controlled by the electroablation module 121 to perform electroablation, allowing the electroablation products to diffuse within the light frozen zone 210, ultimately achieving electrochemical ablation of the tumour at the vascular junction.

Similarly, tumours within cavities present challenges due to the tortuous nature of the cavity walls. Rigid ablation probes cannot be directly inserted through the cavity to the tumour site. Variable ablation probes must therefore traverse the cavity. Typically lacking puncture capabilities, once positioned at the tumour site, the freezing module 112 cools the flexible probe, causing it to adhere to the tumour margin. This achieves relative fixation between the ablation probe and the tumour.

Specifically, the frozen zone 112 employs one or a combination of refrigeration methods including phase-change cooling, compressed gas refrigeration, semiconductor cooling, or refrigerant cooling. The module achieves temperatures below 0°C to cool the ablation probe with freezing function, with multiple refrigeration approaches adaptable to diverse ablation scenarios.

Specifically, the electroablation module 121 incorporates one or a combination of the following: an electrochemical generator, an electrical pulse generator, a pulsed electrolysis generator, a tumour treatment electric field generator, an alternating electric field generator, a radiofrequency generator, a microwave generator, and an electrochemotherapy generator. The radiofrequency generator employs both positive and negative ablation probes; the microwave generator utilises a single electrode-equipped ablation probe. Both generators emit electromagnetic fields that act upon tumour cell membranes, disrupting the potential difference across the membrane. This alters the membrane's permeability to particles, inducing biological effects. The electrochemotherapy generator functions as an electrical pulse generator for pulsed ablation, rupturing cancer cells before delivering chemotherapeutic agents to the target tissue 240 for electrochemotherapy ablation. During actual surgical procedures, when performing electroablation on the target tissue 240, the ablation module 121 may be employed individually or in combination. This enables multiple therapeutic modalities to be applied to the target tissue 240 within a single operation, thereby facilitating tumour ablation.

Furthermore, the ablation system 100 further comprises an ECG R-wave synchronisation module connected to the control module 150. This module acquires the patient's electrocardiogram via ECG electrodes and calculates the R-wave period, thereby controlling the electroablation module 121 to deliver ablation energy exclusively during the R-wave refractory period. During surgery, particularly when ablating tumours near the heart, the cardiac tissue is affected regardless of the magnitude of electrical energy within the ablation module 121. This is especially true for electrical pulse ablation, which delivers extremely high currents within an instantaneous timeframe, thereby generating substantial voltage. During the relative refractory period (RRP), the human body 250 remains highly responsive to such intense electrical stimulation. Consequently, electrical pulse ablation necessitates delivering high-intensity pulses during myocardial refractory periods to prevent stress reactions in the human body 250 following exposure to high voltage. Furthermore, outside the absolute refractory period, it may be employed in conjunction with an electrochemistry generator, tumour therapy electric field generator, alternating electric field generator, or combinations thereof. Electrochemistry ablation using high voltage and high current may also be applied during the cardiac R-wave refractory period. Electrochemical ablation employs direct current, enabling the electroablation module 121 to continuously perform electroablation on the target tissue 240 through diverse methods. This maximises utilisation throughout the entire ablation period, achieving both electroporation and electrochemical ablation of target tissue 240 cells within a shorter timeframe during the same ablation duration. Consequently, electroablation efficiency is enhanced while ablation time is reduced.

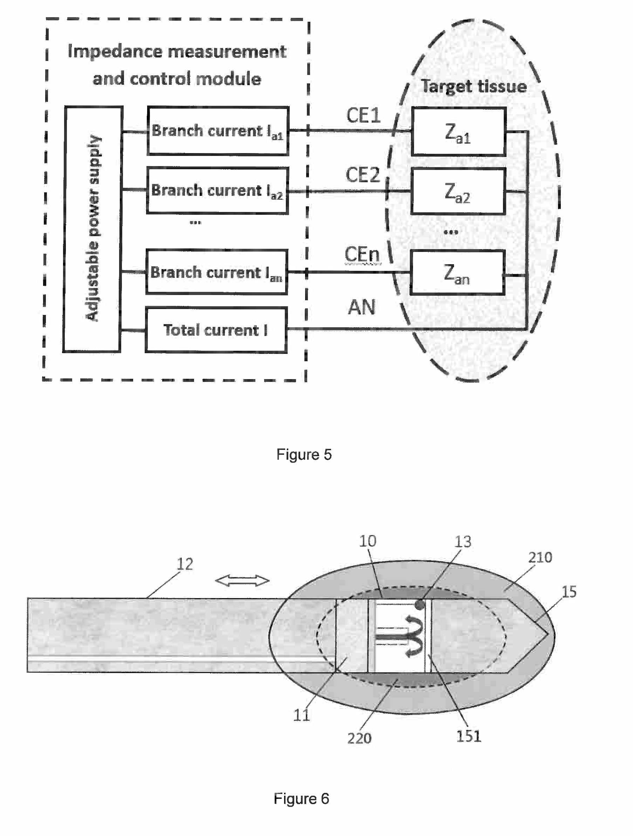

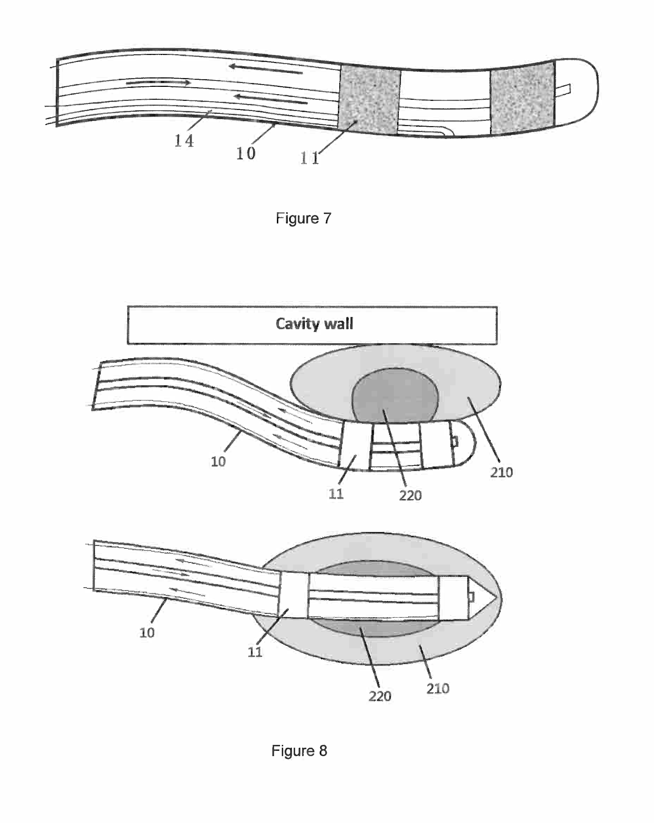

As illustrated in Figures 6 and 7, the present invention further provides an ablation probe comprising a probe shaft 10, at least one electrode 11, and connecting wires. The electrode 11 is connected to the electroablation module 121 via the connecting wires. The electrode 11 is positioned on the probe shaft 10, with the connecting wires linked to the electrode. An electrical insulation layer 12 is provided on the non-electrode zone of the probe shaft 10. Following the formation of the frozen zone 200, at least one electrode 11 is partially positioned within the light frozen zone 210. This arrangement ensures that electroablation can continuously occur within the light frozen zone 210 after the frozen zone 200 has formed. When the frozen zone 200 reaches its maximum extent, tumour cells within the light frozen zone 210 are ablated, thereby preventing the survival of tumour cells in the outer periphery.

Specifically, the electrical insulation layer 12 comprises a biocompatible electrically insulation material, such as an insulation rubber or plastic coating or film layer. Examples include coatings or films of polyethylene terephthalate (PET), Teflon, or polyimide (PI). The probe shaft 10 connects to the frozen zone 112 via a temperature medium delivery tube, which conveys either a cryogenic or thermal medium.

Furthermore, as illustrated in Figure 8, the probe rod 10 and the connecting wire form an integral flexible assembly. When the probe rod 10 is rigid, its tip is pointed; when the probe rod 10 is entirely flexible, its tip is rounded. The variable probe rod 10 is connected to a flexible refrigerant inlet pipe, i.e., the temperature medium delivery pipe, which conveys either refrigerant or heat transfer fluid. The non-electrode zone of the probe rod 10 is provided with an electrical insulation layer 12. Specifically, the temperature medium delivery tube connects to the probe rod 10 and the frozen zone 112. For tumours near vascular structures, the variable probe shaft 10 is guided to the tumour site. By positioning the probe shaft 10 adjacent to the tumour, it cools the tumour to form a frozen zone 200. The tumour tissue near the probe shaft 10 forms a partial deep-frozen zone 220, with the remainder constituting a light frozen zone 210. Electroablation of the light frozen zone 210 is performed via electrode 11. A puncture head is fitted to the variable probe rod 10, which is inserted into the tumour. The variable probe rod 10 then cools the tumour to form frozen zone 200. Electroablation of the tumour is conducted via electrode 11 located within the light frozen zone 210, prioritizing ablation of the tumour's outer periphery for more thorough ablation.

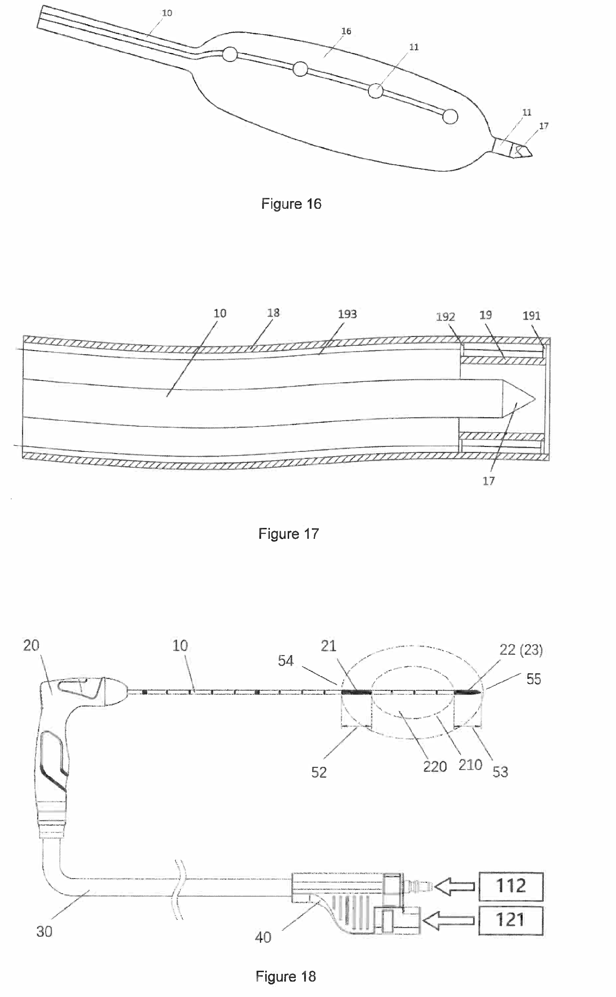

As illustrated in Figure 17, when the probe shaft 10 is entirely flexible, its terminal end features a pointed tip. The probe shaft 10's tip is connected to a puncture head 17. The probe shaft 10 is externally sheathed by a flexible sheath tube 18, which is constructed from insulation material. The probe shaft 10 operatively drives the puncture head to extend or retract within the flexible sheath tube 18. A serpentine tube 19 is positioned at the inner end of the flexible sheath tube 18, with both ends of the serpentine tube 19 fixedly connected to the flexible sheath tube 18 via a first fixing ring 191 and a second fixing ring 192 respectively. The first fixing ring 191 is positioned distally from the end of the flexible sheath tube 18, while the second fixing ring 192 is positioned proximally to the end of the flexible sheath tube 18. A drive wire 193 passes through the first fixing ring 191 and is fixedly connected to the second fixing ring 192. The drive wires 193 are positioned between the serpentine tube 19 and the flexible sheath tube 18. Four drive wires 193 may be arranged uniformly around the serpentine tube 19. During ablation of tumours within the cavity, the drive wires 193 pull the corrugated tube 19, causing it to rotate in the direction of pull. This redirects the tip of the flexible sheath tube, guiding the probe shaft and puncture tip through the sheath to the target area. The probe shaft is then advanced, inserting the puncture tip into the target tissue 240. Upon completion of ablation, the puncture tip is retracted into the flexible sheath tube, which provides protective coverage for the tip. This allows the flexible probe shaft to carry the puncture tip during insertion into the body cavity.

Furthermore, as illustrated in Figure 6, the electrical insulation layer 12 may be slidably mounted upon the probe rod 10, with the electrode 11 positioned externally to the electrical insulation layer 12. By adjusting the position of the electrical insulation layer 12 along the probe shaft 10, the effective area of the electrode 11 is regulated, ensuring the electrode 11 resides within the light frozen zone 210. Following ice ball formation, the electrode 11 and the electrocautery probe establish an electrical pathway within the light frozen zone 210. The electroablation module 121 then prioritizes electrocautery within the light frozen zone 210. Following completion of ablation within the light frozen zone 210, ablation proceeds to the deep-frozen zone 220. During thawing of the target tissue 240, as the ice ball diminishes in size through melting, the electrode 11 on the electrical insulation layer 12 is adjusted by sliding the insulation layer. This ensures the electrode 11 on both the insulation layer and the extension section 15 remains consistently positioned within the light frozen zone 210, preventing electrode exposure beyond the ice sphere and ensuring electroablation consistently occurs within the light frozen zone 210. Adjusting the position of the insulation layer 12 facilitates precise control of electrode 11 positioning within the light frozen zone 210, thereby optimising electroablation efficacy within this zone.

Similarly, the electrical insulation layer 12 may be slidably mounted on the probe shaft 10, with the electrode 11 positioned on the probe shaft 10. By adjusting the position of the electrical insulation layer 12 along the probe shaft 10, the exposed area of the electrode 11 can be modified.

Specifically, the semi-major axis range of the ice sphere formed by freezing the ablation probe is defined as A, while the semi-major axis range of the -21°C isotherm within the ice sphere is defined as B. The end of the electrical insulation layer 12 is positioned on the probe shaft 10 within the interval A minus B. A thermally insulated vacuum sleeve is installed within the probe shaft 10. The distance from the end of the vacuum sleeve within the probe shaft 10 to the end of the probe shaft 10 is set as X. The distance range of the electrical insulation layer 12 from the end of the probe shaft 10 is set between (X+5) and (X+15) cm.

The distance range between the vacuum sleeve and the end of the probe rod 10 is 10 to 40 cm. In this embodiment, the distance between the vacuum sleeve and the end of the probe rod 10 is set to 22 cm, with the edge of the electrical insulation layer positioned at a distance of 27 to 37 cm from the end of the probe rod 10.

Furthermore, the electrical insulation layer 12 is slidably mounted on the probe rod 10. When the ice ball formed by the frozen zone 112, operating within a temperature range below -21°C, extends to cover the electrical insulation layer 12, and when electroablation of the ice ball's periphery is required, the position of the electrical insulation layer 12 can be adjusted. This is achieved by thawing the ice ball, repositioning the electrical insulation layer 12 on the probe rod 10, and then refreezing it. This eliminates the need for probe removal and replacement, simplifying the procedure.

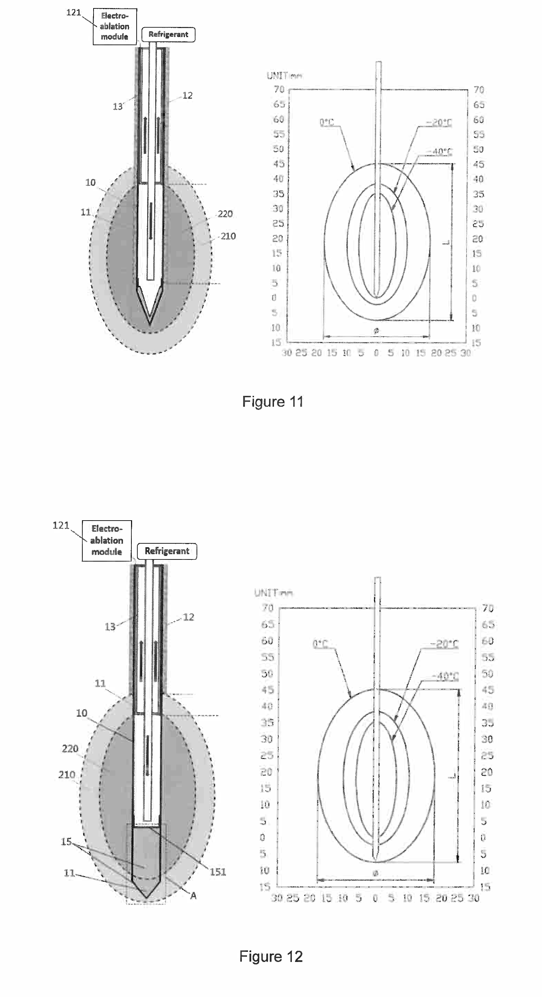

As illustrated in Figure 11, conventional ablation probes require grinding a recess at the tail end of the puncture head during fabrication. This ensures the cannula approaches the probe tip as closely as possible, thereby guaranteeing the tip remains enclosed within the frozen zone 220. However, this recess compromises the integrity of the welded seal between the puncture head and probe shaft. Furthermore, grinding the probe tip risks perforating the puncture head. Alternatively, when the piercing head is fabricated by wrapping a metal sheet around the shaft end for welding, poor weld sealing frequently occurs. Furthermore, when the piercing head material is platinum or platinum-iridium alloy, machining the recess presents significant processing difficulties.

In conventional ablation probes, to position the conductive zone of the probe shaft 10 within the frozen zone 220, the electrical insulation layer completely covers the thermal insulation layer. Upon thawing of the frozen zone 200, the electroablation products diffuse outward from the centre. This results in a large outer perimeter of the target tissue 240, and the thawed frozen zone 200 shrinks to a smaller area. This diffusion of electroablation products beyond the target tissue 240 may result in insufficient dose delivery, leading to incomplete ablation of the outer periphery of target tissue 240 and increased likelihood of tumour recurrence. Furthermore, with conventional ablation probes, once the ice ball is fully formed, the distance between the probe tip and the ice ball's edge is approximately 1 micrometre. During probe insertion, it is difficult for the physician to visually assess the insertion depth via imaging, making it challenging to determine whether the ice ball formed after the probe enters the target tissue 240 adequately encapsulates it.

Furthermore, as illustrated in Figure 12, the ablation probe connects to the frozen zone 112, endowing the probe shaft 10 with freezing functionality. The tip of the probe shaft features an enclosed extension 15, which houses an electrode 11. This electrode 11 within the extension 15 remains partially situated within the light frozen zone 210 at all times. The extension portion 15 is provided with an electrolytic corrosion-resistant isolation section 151 at least near the end of the probe shaft 10 adjacent to the freezing chamber. The isolation section 151 separates the probe shaft 10 from the extension portion 15.

The isolation section 151 blocks the freezing chamber of the probe rod 10, preventing refrigerant from reaching the interior of the extension section 15. The extension section 15 is either solid or hollow in structure, as shown in the enlarged views of the extension section in Figures 13a and 13b. This allows low-temperature energy from the freezing chamber to be conducted through the isolation section 151 and the extension section 15 to the tip. Due to the attenuation of cold energy diffusion, until equilibrium with body temperature is achieved. The extension 15 is positioned such that its tip remains close to the periphery of the ice ball, ensuring that the electrode on the extension 15 remains partially within the light frozen zone 210 regardless of the ice ball's size. Electrocautery can be performed throughout the entire freezing process via the electrode on the extension 15. Once the ice ball is fully formed, electrocautery can be applied to the maximum extent of the light frozen zone 210, yielding superior ablation results around the tumour periphery. The insulation section 151 may be fabricated from platinum or a platinum-iridium alloy. During electrochemical ablation reactions occurring at the extension section 15, the insulation section 151 remains unaffected by electrolysis, thereby safeguarding the probe shaft 10 and preventing gas leakage from the probe shaft 10 during electrochemical reactions.

Furthermore, the ablation probe comprises at least two electrodes 11 connected to the electroablation module 121, mutually insulated and of opposite polarity. One electrode is situated within the light frozen zone or a deep-frozen zone convertible to a light frozen zone, while the other electrode is positioned within the frozen zone or within human tissue or in electrical contact with human tissue. Specifically, one electrode 11 is mounted on the extension portion 15 and located within the light frozen zone 210, while the other electrode is positioned on the rear portion of the probe shaft. The electrode situated outside the probe shaft 10 is located within the deep-frozen zone 220. A conductive pathway 230 is formed by thawing the frozen zone 200 surrounding the probe shaft 10 to the temperature of the light frozen zone 210. This enables electrical conduction between the electrode in the deep-frozen zone 220 of the probe shaft and the light frozen zone 210 via the conductive pathway 230, thereby performing electroablation on the light frozen zone 210. The ablation probe connects to the frozen zone via a connecting tube, enabling cryogenic functionality in either the probe shaft or the balloon. The probe shaft 10 further incorporates a first insulation layer 13. Specifically, the first insulation layer 13 is a vacuum insulation tube. This layer may be positioned internally within the probe shaft 10 or situated between the electrical insulation layer 12 and the probe shaft 10. The periphery of the first insulation layer 13 protrudes beyond the electrical insulation layer 12, exposing the frozen electrode portion within the light frozen zone 210 to maintain electrical conductivity. The distance D1 between the electrical insulation layer 12 and the end of the probe rod 10 is greater than the distance D2 between the first insulation layer 13 and the end of the probe rod 10. The frozen zone 200 of the probe rod 10 and the electrode 11 are located at its front end, wherein the electrode 11 covers the frozen zone 200 of the probe rod 10 and partially extends into the light frozen zone 210. As the rear edge of the ice ball conducts heat through the probe rod, the freezing force is reduced, creating a freezing trail. The difference in protrusion between the thermal insulation layer edge and the electrical insulation layer edge (the difference between D1 and D2) ranges from 1 to 10 mm.

The electrical insulation layer of conventional ablation probes covers the thermal insulation layer. With a probe shaft tip length of 4-6 mm, all electrodes on such probes reside entirely within the deep-frozen zone 220. During freezing module 112 freezing, this prevents both electroablation and cryoablation of the outer periphery of target tissue 240. Electroablation can only occur once the target tissue 240 thaws, and the thawed frozen zone 200 consequently shrinks. This results in incomplete ablation of the outer periphery of the target tissue 240, increasing the likelihood of tumour recurrence.

Extending the probe rod tip to form an elongated section-i.e., integrally extending the puncture head beyond its original configuration-facilitates tip grinding while preventing accidental perforation during the process. This eliminates the need to grind a recess at the puncture head's rear end and allows tip grinding to bypass the probe rod connection point, reducing manufacturing complexity. Additionally, welding the puncture head to the probe rod can avoid the tip area, easing welding difficulty. The electrode at the extended tip of the probe rod may employ platinum-iridium or platinum-iridium coating. Specifically, the drive probe rod tip extends 4-6 mm, with the extension section protruding 3-15 mm beyond the conventional probe rod tip. Furthermore, during probe insertion, physicians may directly advance the tip of the extension section 15 to the tumour margin. Once the cryoballot is formed, it completely encapsulates the tumour, enabling physicians to more readily assess the extent of cryoballot coverage over the tumour during probe placement.

As illustrated in Figure 12, the present invention incorporates a configuration wherein the protrusion of the thermal insulation layer's edge beyond the electrical insulation layer's edge spans 1 to 10 millimetres. Furthermore, the probe rod's tip features an integrally formed extension of 3 to 10 millimetres beyond the conventional probe tip. This arrangement ensures both electrodes at the probe rod's ends remain within the light frozen zone 210. Owing to the characteristics of ice ball formation, the probe rod tip remains consistently proximate to the periphery of the ice ball. This ensures the electrodes on the extension remain perpetually within the light frozen zone 210. Whilst the freezing module 112 performs cryotherapy, the tip of the extension can define the periphery of the formed ice ball, thereby establishing the frozen zone 200 encompassing the tumour. The electroablation module 121 performs electroablation within the light frozen zone 210, maximising the coverage of electroablation products across this zone. This enables thorough electroablation of the outer periphery of the target tissue 240. The puncture tip of the ablation probe functions as an anode in the electrochemical process; electrochemical corrosion of the tip does not compromise the shaft seal integrity of the cryoelectric ablation probe. The cryoelectric probe 131 may be employed as a single unit for both cryoablation and electroablation, replacing the need for separate ablation probes with freezing function and electroablation probes. This reduces the number of probe insertions within the tumour, thereby lowering the complexity of the insertion procedure for the physician. Furthermore, during manufacturing, the cryoelectric probe can be designed with a puncture tip diameter larger than the probe shaft diameter. This ensures the insulated-coated probe shaft and puncture tip share the same diameter, preventing stalling during probe insertion caused by surface steps.

The electrical insulation layer 12 may also extend to cover the extension section 15. During operation, the extension section 15 functions as an electrode, concentrating electrochemical products at the probe shaft tip. Furthermore, the extension section 15 may be fabricated from stainless steel to serve as a cathode, thereby simplifying manufacturing processes, reducing costs, and preventing gas leakage caused by electrochemical corrosion of the probe shaft. The probe shaft 10 and extension 15 may also be fabricated from platinum or platinum-iridium alloy, allowing continuous electrification of the electrode 11 to prevent corrosion of the probe shaft 10 and extension 15.

The electrical insulation layer 12 is slidably mounted on the probe rod 10. The electrode 11 may be positioned externally on the insulation layer 12 or formed as a segment on the probe rod 10. By adjusting the position of the insulation layer 12 along the probe rod 10, the active zone of the electrode 11 can be regulated. As the ice ball thaws and gradually diminishes in size, sliding the electrical insulation layer adjusts the electrode 11 positioned upon it. This ensures the electrode 11 on both the electrical insulation layer 12 and the extension section 15 remains consistently located within the light frozen zone 210. This prevents the electrode from becoming exposed outside the ice ball, thereby ensuring electroablation consistently occurs within the light frozen zone 210.

Specifically, electrode 11 is situated within the frozen zone 200 of freezing module 112. Prior to electroablation, freezing module 112 freezes target tissue 240, enabling the electroablation module 121 to perform electroablation whilst positioned within the ice sphere formed by freezing module 112. The differential protrusion of the thermal insulation layer's edge beyond the electrical insulation layer's edge ranges between 1 and 10 millimetres, ensuring that a portion of electrode 11 resides within the light frozen zone 210. Within this zone, the ice sphere exhibits conductive properties, and the electrical impedance is lower than that outside the ice sphere or within the deep-frozen zone 220. Electroablation performed within the light frozen zone 210 yields superior efficacy, acting exclusively upon the target tissue 240 and delivering a more precisely defined ablation field.

Furthermore, the tip and/or interior of the probe rod 10 may incorporate a temperature detection unit, which may be a thermocouple. This temperature detection unit is positioned at the tip location either internally or externally within the probe rod 10. The temperature detection unit is capable of measuring the temperature of the target tissue 240 at the ablation probe position. Based on the measured temperature, it controls the freezing range and freezing temperature of the freezing module 112, thereby maintaining the temperature within the deep-frozen zone 220 surrounding the cryoablation probe between -21°C and 0°C. This transforms the area surrounding the cryoablation probe within the deep-frozen zone 220 into a light frozen zone 210. Consequently, the temperature-controlled electrodes of the cryoablation probe remain continuously within the light frozen zone 210 and maintain electrical conductivity with the exterior of the ice ball, facilitating electroablation of the external zones of the target tissue 240.

As current ablation probes employ stainless steel, defined by its primary characteristics of rust resistance and corrosion resistance, containing at least 10.5% chromium and no more than 1.2% carbon, the stainless steel probe undergoes electrolysis during electrically powered ablation. The chromium present in the stainless steel is also subject to electrolysis. The normal chromium content within the human body is 6-7 mg, primarily located in bones, skin, and adipose tissue. Electrolysis of the stainless-steel ablation probe can lead to excessive chromium levels within the body, and may also produce hexavalent chromium through electrolysis. Hexavalent chromium is a toxic substance that can adversely affect the human body.

As illustrated in Figure 7, a conduit 14 is installed within the probe shaft 10. One end of conduit 14 is inserted into the interior of probe shaft 10 and is sealed and fixedly connected to the inner wall of probe shaft 10 at the electrode zone. A porous structure is formed at the position of the probe shaft corresponding to the passage tube to vent gases generated by electrochemical reactions. The holes on the probe shaft may be circular, triangular, elongated, or other shapes. The other end of the passage tube is located externally. Furthermore, the external end of the passage tube is connected to an extraction device, which may be selected from an extraction machine, a liquid extraction machine, a vacuum pump, a syringe, or an infusion pump. During electroablation within the cavity, gas generated by the procedure may accumulate, potentially forming an air embolism that poses a lethal risk to the patient. Consequently, a vacuum pump extracts gas from the conduit 14 during electroablation, establishing negative pressure within it. Gases within the cavity are drawn into the conduit 14, whilst gases generated by electrolysis are expelled from the body via the conduit 14. This prevents the accumulation of gases within the patient's body, thereby reducing the likelihood of air embolism. During ablation, conduits 14 may also deliver pharmaceutical agents-such as chemotherapeutic drugs, biological agents, or immunomodulators-to target tissue 240. These agents may be electrically charged; upon injection, they diffuse towards the electrode, coating target tissue 240 to enhance tumour ablation efficacy.

In this embodiment, the conduit 14 may alternatively be positioned externally to the probe shaft 10. A groove may be formed in the outer wall of the probe shaft 10, with the conduit 14 housed within this groove. Following placement in the groove, the conduit 14 is secured via welding. The terminal end of the conduit 14 incorporates a porous structure.

During endoscopic tumour ablation procedures, such as for haemangiomas, tracheal tumours, or lung cancer, the tortuous nature of the endoscopic pathway prevents rigid electroablation probes from directly accessing the target area. A variable probe shaft 10 may be employed. Through the variable probe shaft 10 and the temperature medium delivery tube, the probe can navigate bends within the cavity and deliver energy precisely to the target tissue 240. Position the probe shaft 10 externally against the target tissue 240, then deliver the cooling medium through the temperature medium delivery tube to the tip of the probe shaft 10, lowering the temperature of the electroablation probe below 0°C. The electroablation probe adheres to the exterior of target tissue 240. As tumours within cavities typically form adherent to the cavity wall, to prevent cryogenic necrosis of the cavity wall tissue, the cryoelectric probe freezes only a portion of the target tissue 240 below -21°C. The target tissue 240 near the cavity wall is frozen to between -21°C and 0°C, followed by electroablation via the probe. This allows electrochemical products to diffuse within the frozen zone 200. As the ice ball expands, the electrochemical products rapidly diffuse within the ice ball, achieving complete ablation of the entire target tissue 240. Alternatively, a laser knife may be delivered to the target tissue 240 via the probe shaft 10, cutting the edges of the target tissue 240 to facilitate its excision. More preferably, the probe shaft 10 may simultaneously carry an endoscope, providing a visual field for the probe shaft 10 to facilitate insertion and laser cutting. Upon withdrawal of the electroablation probe, the adhered target tissue 240 is simultaneously extracted from the body.

The electrochemical products of the ablation probe's electrode exhibit properties conducive to cell death through iron-mediated mechanisms. The electrochemical corrosion products of the electrode possess beneficial characteristics for ablation: specifically, they comprise iron or iron-magnesium alloy materials, either individually or in combination. The probe shaft 10, upon energisation of the probe shaft 10, the shaft and its constituent material undergo electrochemical reactions. The electrode at the probe tip 10 is configured as the anode, typically composed of iron, iron alloy, or iron-magnesium alloy. During electrochemical ablation, this anode undergoes sacrificial corrosion. Beyond acidic or alkaline byproducts, the electrochemical reaction yields ferrous ions (Fe²⁺) which diffuse into surrounding tissue and cells, thereby eliminating cancerous cells. Electrolytic products contain iron and magnesium ions. Under the action of ferrous ions or ester oxygenase, these catalyse lipid peroxidation in unsaturated fatty acids highly expressed on cell membranes, thereby inducing ferroptosis and eliminating tumour cells. Furthermore, iron and magnesium ions are essential elements for the human body and are ultimately absorbed without adverse effects. Combining electroablation with cryotherapy, iron ion diffusion concentrates within the light frozen zone 210, enhancing ablation efficacy across the maximum extent of this zone.

Preferably, the ablation probe incorporates an electroablation module 121, with the electrode functioning as the anode or higher potential electrode. The electrode material comprises the probe shaft itself, its outer wall material, cladding, or coating. The electrode material composition is as follows:
The protective outer wall material, cladding, or coating of the electrode is positioned on the probe shaft to participate in electrochemical reactions for consumption. By sacrificing the protective cladding, the probe shaft is safeguarded.

Possessing electrochemical corrosion resistance: specifically comprising one or a combination of platinum, platinum group alloys, graphite, graphene, or carbon fibre materials. When energised, the probe shafts 10 and 10 do not participate in electrochemical reactions, thereby preventing electrochemical corrosion of the probe shafts 10 and 10;
Alternatively, the electrochemical corrosion products exhibit non-toxic properties: specifically, one or a combination of titanium alloys, magnesium alloys, zinc alloys, or optionally a binary Fe-Zn alloy. Upon electrification, the materials of the probe rods undergo electrochemical reactions, yielding electrolytic products that are absorbable by the human body without causing harm. Specifically, this refers to iron alloys that do not contain chromium, as chromium undergoes electrochemical decomposition. Excessive absorption of chromium by the human body may cause harm.

Materials whose electrochemical corrosion products exhibit beneficial properties for tissue dissolution: specifically, iron or iron alloys, which induce ferroptosis in cells.

As illustrated in Figure 15, an ablation probe comprises a probe shaft 10, a balloon 16, at least one electrode 11, and connecting wires. The balloon 16 is mounted on the probe shaft 10 and connected to the end of the rigid or variable probe shaft 10. The balloon constitutes prior art, and its methods of cooling and inflation shall not be elaborated upon herein. Electrode 11 connects to electroablation module 121 via the connecting wire. Positioning electrode 11 on balloon 16, the non-electrode zone of balloon 16 features an electrical insulation layer. At least one electrode 11 is partially arranged within the light frozen zone 210. Electrodes 11 are multiple in number; in this embodiment, four electrodes 11 are provided. Electrode 11 is positioned along the axis of probe shaft 10 to maximise overlap with frozen zone 200, thereby broadening the range of electrochemical products generated during electrochemical ablation. A protective layer, potentially composed of polyamide or polytetrafluoroethylene (PTFE), is applied to the balloon. The variable probe shaft 10 is inserted into a lumen (such as a blood vessel). The balloon 16 distends the tumour within the vessel. Pulsed electricity is applied to the electrodes 11, inducing irreversible electroporation in tumour cells, thereby rupturing their cell walls. The balloon 16 may be a freezing balloon, which freezes the target tissue 240 to form the frozen zone 200. The electroablation module 121 performs electrochemical ablation via electrode 11. Electrochemical by-products can directly penetrate cell walls to eliminate tumour cells, achieving more thorough tumour ablation.

Furthermore, as illustrated in Figure 16, the probe shaft tip 10 may additionally incorporate a puncture head 17, which may also feature electrodes.

Moreover, the ablation probe further comprises a flexible sheath tube 18, which is mounted externally on the probe shaft. The probe shaft is flexible and operatively drives the puncture head to extend or retract within the flexible sheath tube;
A corrugated tube 19 is positioned at the inner end of the flexible sheath tube, with both ends of the corrugated tube fixedly connected to the flexible sheath tube via a first fixing ring 191 and a second fixing ring 192 respectively;
A drive wire 193, which passes through the first fixing ring and is fixedly connected to the second fixing ring, positioned between the corrugated tube and the flexible sheath.

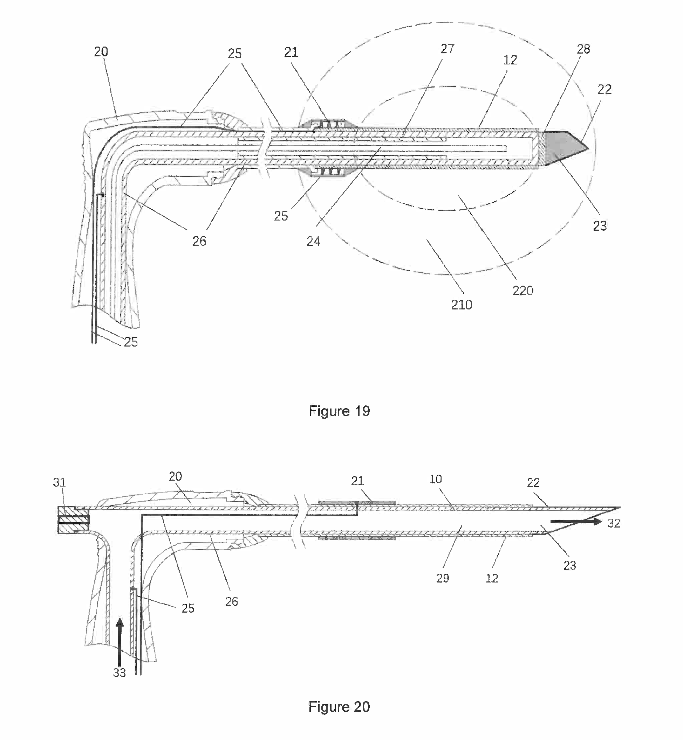

During endoscopic tumour ablation, the probe shaft 10 is guided to the tumour site via the flexible sheath tube and corrugated tube. The probe shaft 10 penetrates the tumour through the puncture tip 17. Subsequently, the balloon 16 is inflated, whereupon the balloon 16 cryopreserves the tumour to form a light frozen zone 210. Finally, electroablation is performed via the electrodes on the balloon 16 and the puncture tip 17. The puncture tip 17 not only enhances the fixation strength between the probe shaft 10 and the tumour but also generates electrolytic products to achieve electrochemical ablation within the tumour, thereby further improving ablation efficacy for tumours within the cavity.
Optionally, as illustrated in Figures 18 and 19, the probe rod 10 comprises a frozen zone and is provided with at least two electrodes of opposite polarity: a first electrode 21 and a second electrode 22. The light frozen zone 210 comprise a first light frozen zone 52 located at the proximal end 54 of the frozen zone 200 and a second light frozen zone 53 located at the distal end 55 of the frozen zone 200., wherein the first electrode 21 is axially positioned within the first light frozen zone 52, and the second electrode 22 is axially positioned at the probe tip of the second light frozen zone 53;
The first electrode 21 and the second electrode 22 are respectively connected to the two oppositely polarised output terminals of the electroablation module 121. The polarity and electroablation parameters of the first electrode 21 and the second electrode 22 are configured by the electroablation module;

An electrical insulation layer 12 is provided in the zone between the first electrode 21 and the second electrode 22, as well as in the non-electrode zones of the probe shaft 10. The insulation strength of the electrical insulation layer is not less than the power supply voltage of the cryoelectric ablation device and the maximum voltage generated from the electroablation module; This is because should the power supply voltage of the cryoelectric ablation device or the voltage output from the electroablation module exceed the insulation strength of the electrical insulation layer installed between the first electrode 21 and second electrode 22 of the probe shaft 10 and in the non-electrode zone, insufficient insulation during operation may cause substantial current to flow into the human body, posing a severe electrocution hazard. Therefore, the insulation strength of the electrical insulation layer must be greater than or equal to both the voltage output from the device's electroablation module itself and the power supply voltage.

Optionally, the axial length of the electrode may be made as equal as possible to the axial length of the light frozen zone.

Optionally, the probe shaft 10 is preferably fabricated from conductive material to establish electrical connectivity between the first electrode 21 or second electrode 22 and the electroablation module 121.

Optionally, the probe shaft 10 may feature an exposed portion functioning as an electrode.

Optionally, the probe shaft 10 may be a rigid probe shaft or a bendable variable probe shaft.

Optionally, as shown in Figure 18, the ablation probe further comprises a probe handle 20, a connecting tube 30, and a connecting head 40. One end of the probe shaft 10 is fixedly connected to the probe handle 20, and the connecting head 40 is connected to the lower end of the probe handle 20 via the connecting tube 30.

Optionally, as shown in Figure 19, a second insulation layer 28 is provided between the probe tip 23 and the remaining portion of the probe shaft 10. Said second insulation layer 28 is fabricated from vacuum, plastic, or other insulation materials to ensure that at least one second electrode 22 on the probe tip remains within the light frozen zone 210 and thus conductive. An outer insulation layer 27 is provided on the outer wall 26 of the probe shaft 10, fabricated from vacuum, plastic, or other insulation materials to ensure normal tissue remains undamaged during cryotherapy; An electrical insulation layer 12 is positioned externally on the probe rod insulation layer 27. Controlled by the frozen zone 112, refrigerant is conveyed through the refrigerant pipe 24 to the target zone 240 for freezing, forming a deep-frozen zone 220 and a light frozen zone 210. The polarity and electroablation parameters of the first electrode 21 and second electrode 22 are set via the electrochemical module 121. A predetermined current is delivered to the target area 240 through the electrode connection wires 25 (e.g., enameled wire, plastic with metal plating) to perform electroablation. This positions the second electrode 22 within the light frozen zone.

Optionally, as illustrated in Figure 20, the probe shaft 10 comprises a hollow tube internally, with the probe tip 23 featuring an outlet port 32 for dispensing refrigerant or medication. The end of the probe shaft remote from the probe tip 23 incorporates an injection port 31, enabling medication injection via this port and subsequent dispensing of refrigerant 33 or medication through the outlet port 32; The electroablation module 121 is configured to adjust and configure the polarity and ablation parameters of the ablation probe electrode based on the polarity of the drug molecules, thereby increasing the penetration speed and range of the drug molecules between the electrodes and within the target tissue.

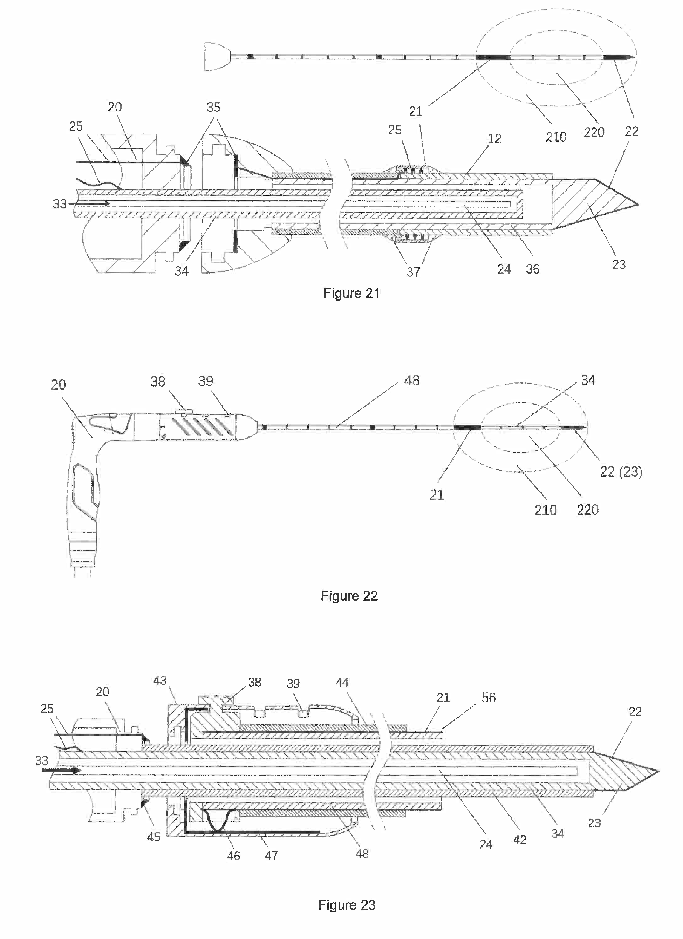

Optionally, as shown in Figure 21, the probe rod 10 is provided with a probe handle 20 at one end; the ablation probe further comprises an outer probe rod 36, which is removably and tightly fitted over the probe rod 10 of the ablation probe, connected to the probe handle 20, and electrically connected to the electroablation module 121; a first electrode 21 is positioned at the proximal end of the first light frozen zone 52 within the freezing zone of the outer probe shaft 36, while a second electrode 22 is situated at the probe tip of the second light frozen zone 53 at the distal end of the freezing zone; the ablation probe functions as an ablation probe with freezing function, electroablation probe, or cryoelectric ablation probe, forming an integral unit with the probe handle 20 and being reusable; the outer probe shaft 36 and ablation probe exhibit excellent thermal and electrical conductivity. The first electrode 21 on the outer probe shaft 36 and the probe handle 20 of the ablation probe are electrically connected via contact spring 35.

Optionally, the first electrode 21 comprises a ring-shaped corrosion-resistant metal (e.g., platinum-iridium ring) or its plated electrode (e.g., platinum-plated), electrically connected to the electrode lead 25 via welding, conductive adhesive, enameled wire winding, printed circuitry, etc. The electrode leads 25 may be enameled wire, printed circuitry, metal plating, metal sheathing, etc. The annular metal electrode 21 and the outer probe rod 36 are sealed with a biocompatible sealing compound 37 between them, facilitating puncture, preventing corrosion, and inhibiting bodily fluid ingress. The second electrode 22 comprises the exposed metal probe tip 23 of the outer probe rod 36, with the probe tip 23 coated with a corrosion-resistant metal or its conductive plating, electrically connected to the electroablation module 121 via the outer wall of the outer probe rod 36.

Optionally, as illustrated in Figures 22 and 23, the ablation probe further comprises an outer sheath 41. Said outer sheath 41 is removably fitted over the probe shaft 10 of the ablation probe, being axially slidable along the probe shaft 10. One end of the outer sheath is connected to the probe handle 20 of the ablation probe. The outer sleeve comprises a rod-shaped portion 48, with a first electrode 21 positioned on said rod-shaped portion 48, and a second electrode 22 positioned at the probe tip 23 of the ablation probe.

The outer casing is provided with a slider 38 and a groove 39, wherein the groove 39 is configured to position the first electrode 21; the slider 38 is fixedly connected to the rod-shaped portion 48, and the slider 38 is configured to drive the rod-shaped portion 48 to slide axially along the probe rod 10, thereby driving the first electrode 21 to move correspondingly. This alters the spacing and impedance between the first electrode 21 and the second electrode 22, ensuring both electrodes are positioned within the light frozen zone 210.

Optionally, the outer casing 41 may be an insulated tube, wherein sliding the outer casing 41 adjusts the lengths of the frozen zone 200 and light frozen zone 210. The first electrode 21 is positioned at the distal end 56 of the insulated tube to ensure the first electrode 21 remains within the light frozen zone 210 of the frozen zone 200.

Optionally, as shown in Figure 23, the first electrode 22 contacts and slides along the spring-loaded rail 47 on the outer sleeve 41 via the spring-loaded plate 46 on the rod-shaped portion 48, subsequently contacting the probe handle spring-loaded plate 45. Upon snap-fastening connection between the outer sleeve 41 and ablation probe, electrical connection between the two is established.

Optionally, as shown in Figure 23, an outer sliding sleeve 43 is further fitted over the outer sheath 41. An outer sliding sleeve insulation layer 44 is provided between the outer sliding sleeve 43 and the first electrode 21 on the latter.

Optionally, as shown in Figure 23, the outer sleeve 41 may additionally incorporate thermal insulation functionality, wherein the rod-shaped section 48 comprises a vacuum insulated wall. Sliding the outer sleeve 41 controls the length and size of the frozen zone 200 and the light frozen zone 210. The first electrode 21 is positioned at the distal end 56 of the rod-shaped section 48 of the outer sleeve 41, ensuring the first electrode 21 remains within the light frozen zone 210 of the frozen zone.

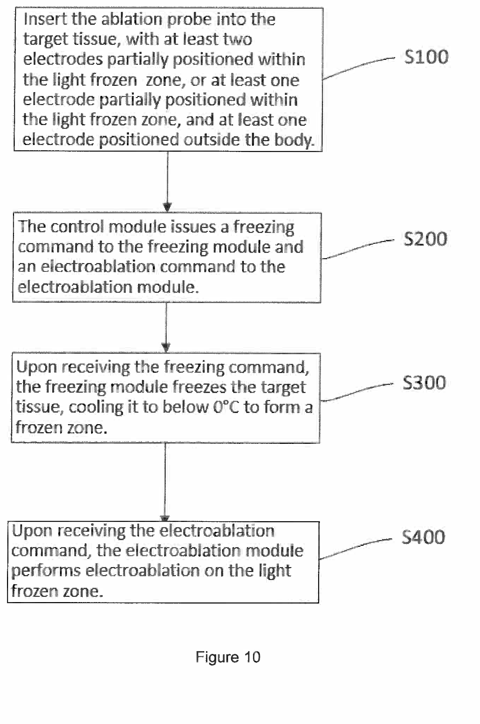

As illustrated in Figure 10, the present invention further provides an ablation control method combining freezing and electroablation, characterised by application within the aforementioned ablation system 100. The ablation control method comprises the following steps:
S100: Inserting the ablation probe into the target tissue 240, wherein at least two electrode portions are arranged within the light frozen zone 210 or at least one electrode portion is arranged within the light frozen zone 210, and at least one electrode of opposite polarity is arranged within the human body tissue 250 or in electrical contact with the human body tissue 250;
Preferably, prior to inserting the ablation probe into the target tissue 240, inject a conductive liquid into the target tissue 240 that is biocompatible, non-harmful to tissue and/or beneficial for ablation. The conductive liquid may be a high-concentration saline solution or a liquid containing iron ions. Prior to insertion of the ablation probe, the conductive fluid is injected into the target tissue 240 via a lumen in the ablation probe or an injection syringe, followed by freezing and electroablation.

In conventional ablation therapy, saline is injected into the target tissue 240 to isolate freezing/thermal effects and prevent damage to normal tissue.

In this patent, the injection of highly concentrated saline solution lowers the freezing point of the target tissue 240. Once the frozen zone 200 is fully formed, this allows for a larger extent of the light frozen zone 210, thereby expanding the effective range of electroablation and enhancing the conductivity within the light frozen zone 210.

Additionally, a solution containing iron ions may be injected. freezing causes cell walls to rupture, and under electroablation, this facilitates the diffusion of iron ions within the target tissue 240, promoting ferroptosis in tumour cells.

S110: Control module 150 issues impedance monitoring commands to impedance measurement and control module 122. This module monitors and displays inter-electrode impedance data while calculating and regulating electroablation parameters;
S120: Upon receiving inter-electrode impedance information, the control module plans electrode distribution, which is then implemented by the operator;
S130: Upon receiving electrode-to-electrode impedance information, the control module plans electrode grouping, which is then implemented by the operator.

The calculation and control of electroablation parameters include:
Uncontrolled electroablation current: current distribution between electrode branches is inversely proportional to impedance;
Equal current distribution: current is distributed equally among all electrode branches;
Impedance-proportional current distribution: lower impedance in an electrode branch corresponds to lower current;
Time-division electroablation: electroablation is performed sequentially in time-division mode using grouped cryoelectric probe 131 and electroablation electrodes;
S200: Controls freezing module 112 or cryo equipment to issue cryo commands, freezing target tissue 240 below 0°C via cryo-capable ablation probes to form frozen zone 200;
Temperature measurement module 111 monitors the target tissue 240's temperature, acquiring temperature signals. Subsequently, control module 150 coordinates the electroablation module 121 and temperature measurement module 111 to operate on the ablation probe in a time-division manner, ensuring mutual non-interference;
Temperature measurement module 111 transmits temperature readings to control module 150, which upon receiving these values regulates the operational status of freezing module 112 and electroablation module 121;
S210: The control module 150 issues a thawing command to the freezing module 112. Upon receiving this command, the freezing module thaws the ablation probe with freezing function or cryoelectric probe electrode's contact area within the frozen zone 200 to the temperature of the surrounding light frozen zone 210. This forms a conductive pathway 230 around the probe electrode within the light frozen zone 210;
S220: Upon completion of thawing the frozen zone 200 contacted by the ablation probe with freezing function, a conductive pathway 230 is formed between the electrode in this zone and either the electrode in the light frozen zone 210 or the electrode at the periphery of the frozen zone 200. The electroablation module 121 performs electroablation on the light frozen zone 210 or the outer edge of the frozen zone 200 via a pathway formed by the electrode within the thawed frozen zone 200, the electrode at the outer edge of the frozen zone 200, and the conductive channel 230;
S221: During the process where the electroablation module 121 performs electroablation on the periphery of the frozen zone 200 via a pathway formed by electrodes within the thawed deep-frozen zone 220, electrodes at the periphery of the light frozen zone 210 or frozen zone 200, and the conductive channel 230, the electroablation products are transferred between the conductive channel 230 and the periphery of the light frozen zone 210 or frozen zone 200;
Following transfer of the electroablation products between the conductive channel 230 and the periphery of the light frozen zone 210 or the frozen zone 200, the control module 150 directs the freezing module 112 to reduce temperature. The temperature measurement module 111 monitors the frozen zone 200, maintaining the entire scope of the frozen zone 200 and the scope of the light frozen zone 210;
S230: Upon completion of electroablation within the light frozen zone 210, proceed to ablate the deep frozen zone 220;
S240: The target tissue (muscle or nerve tissue) 240 is frozen using an ablating probe with freezing capability, placing it within the deep-frozen zone 220 to render it non-conductive. This inhibits nerve conduction and muscle contraction, thereby achieving freezing anaesthesia;
S300: Prior to, concurrently with, and/or following freezing, control the electroablation module 121 or electroablation device to issue electroablation commands. Employ the electroablation electrode to perform electroablation first on the light frozen zone 210, then on the deep-frozen zone 220.

Specifically, the temperature of the frozen zone 200 is below 0°C, and the temperature of the frozen zone 200 after thawing is above -21°C.

Ablation of deep-frozen zone 220 comprises:
Control module 150 issues a cooling signal to frozen zone 112, which directly performs freezing on the zone;
Alternatively, the control module 150 transmits an electroablation signal to the electroablation module 121, raising the temperature of the zone above -21°C to perform electroablation.

During cryoanaesthesia via the freezing module 112, the ablation probe is inserted into the nerve or muscle. The freezing module 112 employs the freezing function of the ablation probe to freeze the nerve and muscle tissue to the deep-frozen zone 220 temperature, thereby blocking nerve conduction and muscle contraction, interrupting nerve pathways and/or suppressing tissue activity. During electroablation, should excessive voltage be applied-such as during electrical pulse ablation-the ablation probe positioned within the nerve or muscle tissue is cooled below -21°C. This temporarily renders the muscle and nerve incapable of activity and conduction, thereby mitigating or eliminating muscle and tissue contractions, organ damage, and pain experienced by the patient under electroablation stimulation.

In another embodiment, the ablation probe with freezing function may be positioned centrally within the frozen zone 200, as illustrated in Figure 3. Following freezing of the target tissue 240 within the frozen zone 112, thawing is omitted. Freezing is then directly performed on the light frozen zone 210 using the cryoelectric probe 131 and electroablation probe. Electrochemical products diffuse outward from the target tissue 240, preferentially eliminating cancer cells in the outer periphery of the target tissue 240, while cancer cells within the inner core of the target tissue 240 are ablated via cryotherapy.

Alternatively, the cryoelectric probe may be positioned at the centre of the frozen zone 200. Following freezing of the target tissue 240 by the frozen zone 112, the frozen zone 200 is allowed to thaw naturally. As illustrated in Figure 3, during thawing of the ice sphere, the sphere's overall volume decreases, gradually raising the internal temperature of the entire ice sphere to between -21°C and 0°C before proceeding with electroablation. During cryoablation, the interior of the target tissue 240 is first subjected to cryoablation, followed by electroablation of the entire interior of the ice sphere after its thawing.

Optionally, the cryoelectric probe may be positioned centrally within the frozen zone 200. Following freezing of the target tissue 240 by the freezing module 112, the frozen zone 200 undergoes active thawing, as illustrated in Figure 3. Following thawing of the deep-frozen zone 220, the zone near the outer circumference of the cryoelectric probe 131 thaws preferentially to between -21°C and 0°C. This establishes an electrical pathway between the entire cryoelectric probe 131 and the light frozen zone 210 (the outer periphery of the ice ball). Electroablation is then performed after complete thawing. The expanded area of the cryoelectric probe 131 participating in electrochemical reactions further enhances the electrochemically-assisted ablation efficacy, yielding superior ablation results within the light frozen zone 210.

In another embodiment, the cryoelectric probe may be positioned at the periphery of the light frozen zone 210, as illustrated in Figure 4. Following freezing of the target tissue 240 by the freezing module 112, thawing is not performed. Instead, cryoelectric ablation of the light frozen zone 210 is directly conducted using the cryoelectric probe 131 and electroablation probe. Electrochemical products diffuse outward from the target tissue 240, preferentially eliminating cancer cells in the outer periphery of the target tissue 240, while cancer cells within the inner core of the target tissue 240 are ablated via cryotherapy.

Optionally, the cryoelectric probe may be positioned at the periphery of the light frozen zone 210. Following freezing of the target tissue 240 by the frozen zone 112, the frozen zone 200 undergoes active thawing, as illustrated in Figure 4. Following thawing of the deep-frozen zone 220, the periphery near the cryoelectric probe 131 is preferentially thawed to between -21°C and 0°C. This establishes an electrical pathway between the entire cryoelectric probe 131 and the electrodes of the electroablation probe within the light frozen zone 210. Electroablation commences upon completion of thawing. The expanded area of the cryoelectric probe 131 participating in electrochemical reactions further enhances the electrochemically-assisted ablation efficacy, yielding superior ablation results in the light frozen zone 210.

The aforementioned ablation control method achieves the following technical effects:
1. Confinement of target tissue 240 within the frozen zone 200, with the ablation probe positioned either within the target tissue 240 or immediately adjacent to its exterior. This restricts the electroablation effect to the interior of the frozen zone 200, minimising damage to surrounding areas outside this zone.

The electroablation module 121 performs electroablation via electrodes within the light frozen zone 210, prioritizing ablation within this zone to concentrate and direct the effects of electroablation products within this area.

2. During the thawing process of the frozen zone 200, the ablation probe with freezing function commences heating. This raises the temperature of the frozen zone 200 adjacent to the probe until both the inner core and outer periphery of the frozen zone 200 reach temperatures between -21°C and 0°C. A conductive channel 230 forms between the frozen zone 200 at the probe tip and its outer periphery. This positions the electrodes on the ablation probe within the conductive channel 230. Electroablation will primarily affect the periphery of the frozen zone 200, whilst the target tissue 240 may be ablated either through cryoablation or electroablation.

The temperature of the frozen zone 200 ranges from -196°C to 0°C, wherein the frozen zone 112 may freeze the target tissue 240 to between -21°C and 0°C for cryopreservation and to serve as a conductive carrier for electroablation. The frozen zone 112 may also freeze the target tissue 240 to between -196°C and -21°C to perform freezing on the target tissue 240; as the frozen zone 200 forms an ice ball by freezing the target tissue 240, this ice ball can secure the ablation probe, maintaining a relatively fixed position between the ablation probe and the target tissue 240. This ensures greater precision in the scope of cryoablation and electroablation.

Taking as an example a configuration where one ablation probe with freezing function with an electrode is . positioned at the centre of the frozen zone 200, flanked by two electroablation probes, the frozen zone 112 initially cools the target tissue 240. When the ablation probe with freezing function with an electrode is situated within the deep-frozen zone 220, electrical conductivity is absent between this probe and the electroablation probes. Conversely, electrical conductivity exists between the electroablation probes located within the light frozen zone 210.

The ice ball (frozen zone 200) is divided into zones C and D. Zone A constitutes the central zone of the ice ball, i.e., the frozen zone, while zone D comprises the two terminal zones of the ice ball, i.e., the frozen diffusion zones.

As illustrated in Figure 3, since the electroablation probes are situated within the light frozen zone 210, the conductive pathways 230 formed between these probes lie within the scope of the ice ball's C zone. Consequently, the majority of the electroablation products are deposited upon the target tissue 240 within the C zone.

As illustrated in Figure 3, post-thawing, the temperature at the centre of the frozen zone 200 exceeds -21°C. This enables the formation of a conductive pathway 230 between the electrodes on the ablation probe with freezing function within the deep-frozen zone 220 and those on the electroablation probe within the light frozen zone 210. Consequently, the majority of the electroablation products target the tissue 240 within zone D.

Within the frozen zone 200, electro-dissolution of zone C is performed directly via the two electro-dissolution probes. Subsequently, rewarming establishes the conductive pathway 230 between the deep-frozen zone 220 and light frozen zone 210, enabling the cryoelectric probe 131 and electro-dissolution probe to perform electro-dissolution on zone D. This ultimately achieves complete electro-dissolution of the entire light frozen zone 210, ensuring more thorough electro-dissolution of the outer periphery of the ice ball.

Following rewarming of the deep-frozen zone 220, electroablation was performed for a period to concentrate ablative products within zone D. Target tissue 240 was then refrozen to maintain the extent of the light frozen zone 210, confining the electroablation products within the light frozen zone 210. The central zone of the ice ball remains frozen, preventing the diffusion of electroablation products into its core. This significantly enhances the ablation efficacy of tumour cells at the periphery of the ice ball.

Furthermore, ablation of the deep-frozen zone 220 comprises:
Control module 150 transmits a cooling signal to frozen zone 112, which directly performs freezing on the designated area;
Alternatively, the control module 150 issues an electroablation signal to the electroablation module 121, whereby the zone is heated to above -21°C for electroablation.

The timing of the control module 150 issuing the electroablation signal to the electroablation module 121 occurs before and/or during and/or after the formation of the frozen zone 200.

3. The ablation probe is designed with multiple electrodes on the same probe. A probe tip insulation layer 28 is provided between the probe tip 23 and the remaining portion of the probe shaft 10, enabling the second electrode 22 on the probe tip to remain within the light frozen zone and conductive. An additional probe shaft insulation layer 27 may be provided externally on the outer wall 26 of the probe shaft 10, utilising vacuum, plastic, or other insulation materials, to ensure the second electrode 22 remains within the light frozen zone and conductive.

The ablation probe is fitted with an outer sleeve tube 41, which incorporates a slider and groove. This slider drives the rod-shaped section to slide axially along the ablation probe shaft, thereby causing the first electrode to move correspondingly. This alters the spacing and impedance between the first and second electrodes, ensuring both electrodes reside within the light frozen zone. Sliding the outer sleeve controls the length and size of both the frozen zone and the light frozen zone. During operation, the practitioner inserts the multi-electrode ablation probe, fitted with the outer sleeve 41, into the target tissue area for ablation. Should the ablation zone prove insufficiently large to encapsulate the target area with an ice ball, the practitioner may slide the slider distally to expand the cryopreservation zone, securing it at a groove corresponding to a predetermined distance. Conversely, should the ablation range prove excessive during the ablation process, causing the ice ball to envelop both the target area and surrounding healthy tissue, the user may reduce the frozen zone's extent by sliding the slider distally towards the probe tip. The slider is then secured in a recessed groove at a predetermined distance.

Although preferred embodiments of the present invention have been described, those skilled in the art, once acquainted with the fundamental inventive concept, may make further alterations and modifications to these embodiments. The appended claims are therefore intended to be interpreted as encompassing the preferred embodiments and all alterations and modifications falling within the scope of the present invention.

It will be apparent to those skilled in the art that various modifications and variations may be made to the present invention without departing from the spirit and scope thereof. Thus, if such modifications and variations fall within the scope of the claims and their equivalents, the present invention is intended to encompass them.

## Claims

1. A combined cryoablation and electroablation system, wherein said system comprises a freezing module and an electroablation module;
said freezing and said electroablation modules are connected to ablation probes, which are used to cryoablate and/or electroablate the target tissue;
said freezing module is configured to freeze the target tissue below 0°C to form a frozen zone; said frozen zone is subdivided into a light frozen zone and a deep-frozen zone, wherein the temperature range of said light frozen zone is set at -21°C to 0°C, which is the electrically conductive zone and capable of electroablation; the temperature range of said deep-frozen zone is set below -21°C, which is non-conductive zone and incapable of electroablation;
said electroablation module is connected to at least two electrodes, said electrodes comprise at least one first electrode and at least one second electrode, wherein said first and said second electrodes are mutually insulated and connected to said electroablation module's two output ports of opposite polarity; at least one of said first electrodes is partially placed in said light frozen zone of the target tissue or in a deep-frozen zone convertible to a light frozen zone, while said second electrodes are partially placed in said frozen zone of the target tissue or the human tissue, or in electrical contact with human tissue;
said freezing module and said electroablation module work in conjunction to control the execution of electroablation before cryoablation, and/or concurrently with cryoablation, and/or after cryoablation.

2. The combined cryoablation and electroablation system of claim 1, wherein said freezing module is a separate freezing device, and/or said electroablation module is a separate electroablation device.

3. The combined cryoablation and electroablation system of claim 2, wherein said system further comprises a control module connected to said freezing module and said electroablation module to control their freezing and electroablation operations.

4. The combined cryoablation and electroablation system of claims 1 to 3, wherein said freezing module possesses the thawing function, which can selectively freeze or thaw said ablation probe with freezing function connected thereto, controlling the electrode contact area of said ablation probe in said light frozen zone thereby forming a conductive pathway to achieve electroablation, or controlling the electrode contact area of said ablation probe to be in said deep-frozen zone to prevent electroablation of said electrode, or controlling the magnitude of the electrical impedance between the electrodes of said ablation probe.

5. The combined cryoablation and electroablation system of claim 3 , wherein said system further comprises a temperature measurement module connected to said control module or said freezing module, wherein said temperature measurement module measures the temperature of the target tissue via a separate temperature measurement probe and/or a temperature measurement thermocouple located on said ablation probe, and monitors the extent and temperature of said frozen zone through temperature feedback to control the electrical conductivity of said frozen zone.

6. The combined cryoablation and electroablation system of claims 1 to 3, wherein said electroablation module further comprises an impedance measurement and control module for monitoring the impedance between electrodes and its variation during cryoablation and/or electroablation, which can be used to calculate and control electroablation parameters, determine and adjust the position and distribution of electrodes in the tissue, determine and adjust participating electrodes and their polarity, determine and adjust series or parallel connections and grouping of electrodes, and determine and adjust ablation sequence.

7. The combined cryoablation and electroablation system of claim 6, wherein said impedance measurement and control module comprises voltage and current adjustment and distribution circuits, based on the impedance and its variations monitored between said electrodes, controlling average distribution of voltage and current across the main and branch circuits, proportional distribution to impedance magnitude, or inversely proportional distribution to impedance magnitude, controlling duration and time-division energization of the electroablation, controlling and distributing ablation voltage, current, and ablation energy through electrodes.

8. The combined cryoablation and electroablation system of claim 7, wherein an electrical conductive liquid or drug that is biocompatible, non-harmful to tissue, and/or beneficial to ablation is injected into a selected region of the target tissue to increase conductivity and drug concentration in that zone; or a non-conductive or antifreeze liquid that is biocompatible, non-harmful to tissue, and/or beneficial to ablation is injected to reduce the conductivity and freezing efficacy of the zone.

9. The combined cryoablation and electroablation system of claim 3, wherein said system further comprises an ECG R-wave synchronisation module, said ECG R-wave synchronisation module acquires the patient's electrocardiogram via ECG electrodes and calculates the R-wave period; said control module regulates the electroablation energy of said electroablation module to be delivered exclusively within the R-wave refractory period based on the patient's ECG and R-wave period.

10. The combined cryoablation and electroablation system of claims 1 to 3, wherein said electrodes are positioned within said light frozen zone, and said freezing module is controlled to refrain from actively thawing the target tissue, relying instead on its natural thawing process, thereby prioritising electroablation of said light frozen zone; subsequently, as the target tissue thaws naturally, the temperature of said deep-frozen zone rises to that of said light frozen zone, enabling electroablation of the entire target tissue; the process of freezing-natural thawing-electroablation is repeated.

11. The combined cryoablation and electroablation system of claim 4, wherein said freezing module is controlled to slightly thaw the electrode contact area of said ablation probe with freezing function, raising its temperature to that of said light frozen zone to establish electrical conduction with the target tissue, but not to thaw said deep-frozen zone outside the electrode contact zone of said ablation probe, enabling electroablation of said light frozen zone first; as the target tissue thaws naturally, the temperature of said deep-frozen zone rises to that of the light frozen zone, to subsequently perform electroablation of the entire target tissue; the process of freezing -electrode thawing - electroablation is repeated.

12. The combined cryoablation and electroablation system of claims 1 to 3, wherein during the gradual withdrawal of said ablation probe from the target tissue, the freezing zone or electrode zone of said ablation probe is utilized to perform continuous segmental freezing and/or electroablation of the puncture probe tract.

13. An ablation probe, applicable to said combined cryoablation and electroablation system of claims 1 to 12, comprises a probe shaft, at least one electrode and connecting wire, wherein said electrode is mounted on said probe shaft and connected to said electroablation module via said connecting wire, the non-electrode area of said probe shaft is equipped with an electrical insulation layer, at least one said electrode is partially located within said light frozen zone.

14. An ablation probe, applicable to said combined cryoablation and electroablation system of claims 1 to 12, comprises a probe shaft, a balloon, at least one electrode and connecting wire, wherein said electrode is connected to said electroablation module via said connecting wire, said ballon is connected to the end of said probe shaft, said electrode is mounted on said balloon, the non-electrode area of said balloon is equipped with an electrically insulation layer, at least one said electrode is partially located within said light frozen zone.

15. The ablation probe of claim 13 or 14, when said electrode of said ablation probe is used as an anode or a higher potential electrode, the material of the electrode, its outer wall, cladding, or coating for isolating and protecting said electrode comprises:
materials with resistance to electrochemical corrosion, including one or a combination of platinum, platinum group alloys, graphite, graphene, or carbon fiber materials; or materials whose electrochemical corrosion products exhibit non-toxic properties, including one or a combination of titanium alloys, magnesium or zinc alloys; or materials whose electrochemical corrosion products possess properties beneficial to ablation, including one or a combination of iron or iron alloy materials.

16. The ablation probe of claim 13 or 14, wherein said electrical insulation layer comprises a biocompatible polymeric electrical insulation material, and said electrical insulation layer is a coating or a film layer.

17. The ablation probe of claim 13 or 14, wherein said ablation probe is connected to said freezing module via a connecting tube to enable freezing function in said probe shaft or said balloon, a first thermal insulation layer is fitted with said probe shaft or said balloon, and the edge of said first thermal insulation layer protrudes beyond said electrical insulation layer, so that said electrode is partially situated within said light frozen zone after freezing.

18. The ablation probe of claim 17, wherein the edge of said first thermal insulation layer protrudes to the edge of said electrical insulation layer by a range of 1 to 10 mm.

19. The ablation probe of claim 13, wherein an extension is externally connected to the distal end of the freezing chamber of said probe shaft, with the refrigerant unable to reach said extension, and an electrode is fitted on said extension, controlling the electrode on said extension remained partially within the light frozen zone throughout the freezing process.

20. The ablation probe of claim 19, wherein an electrolytic corrosion-resistant barrier is provided at the junction between said extension and the freezing chamber of said probe shaft, isolating said freezing chamber from said extension.

21. The ablation probe of claim 19, wherein said length of the extension is 3 to 15 mm.

22. The ablation probe of claim 13 or 14, wherein said ablation probe comprises at least two electrodes connected to said electroablation module, mutually insulated with opposite polarity, wherein one electrode is located in said light frozen zone or said deep-frozen zone convertible to said light frozen zone, and said other electrode is located in said frozen zone or within human tissue or in electrical contact with human tissue.

23. The ablation probe of claim 13 or 14, further comprising:
a puncture head connected to the tip of the probe shaft;
a flexible sheath tube, which is fitted over the exterior of the probe shaft, the probe shaft being flexible and operatively driving the puncture tip to extend or retract within the flexible sheath tube;
a corrugated tube positioned at the inner end of said flexible sheath tube, both ends of said corrugated tube being fixedly connected to said flexible sheath tube via a first fixing ring and a second fixing ring respectively;
a driving wire, which passes through said first retaining ring and is fixedly connected to said second retaining ring, with the driving wire positioned between said corrugated tube and said flexible sheath tube.

24. The ablation probe of claim 13 or 14, wherein a conduit is disposed within said probe shaft, one end of said conduit opens at said electrode of the probe shaft and communicates with the target tissue, and the other end of said conduit opens externally.

25. The ablation probe of claim 24, wherein the external end of said conduit is connected to a suction device, a fluid extraction device, a vacuum pump, a syringe, or an infusion pump.

26. The ablation probe of claim 13, wherein said probe shaft comprises a frozen zone and is provided with at least two electrodes of opposite polarities, namely a first electrode and a second electrode; said light frozen zone comprises a first light frozen zone located proximally within said frozen zone and a second light frozen zone located distally within said frozen zone; said first electrode is axially positioned within said first light frozen zone; said second electrode is axially positioned at said probe tip within said second light frozen zone; said first electrode and said second electrode are respectively connected to two oppositely polarised outputs of said electroablation module, with the polarity and electroablation parameters of said first and second electrodes being configured by said electroablation module; electrical insulation layers are provided in the zone between said first and second electrodes and in the non-electrode zone of said probe shaft, with the insulation strength of electrical insulation layer no lower than the power supply voltage of said cryoablation and electroablation system or the maximum voltage generated from said electroablation module.

27. The ablation probe of claim 26, wherein said probe shaft is a rigid probe shaft or a bendable flexible probe shaft.

28. The ablation probe of claim 26, wherein said axial length of the electrode is equal to the axial length of said light frozen zone.

29. The ablation probe of claim 26, wherein said probe shaft is made from electrically conductive material, to establish the electrical connection between said first or second electrodes with the electroablation module.

30. The ablation probe of claim 26, wherein said probe shaft has an exposed portion serving as an electrode.

31. The ablation probe of claim 26, wherein a second thermal insulation layer is provided between the tip and the remaining portion of said probe shaft, formed from vacuum, plastic or other thermal insulation material, to make said second electrode within said light frozen zone.

32. The ablation probe of claim 26, where said probe shaft is hollow inside, and its tip comprises an outlet for dispensing refrigerant or drugs, and said electroablation module is configured to adjust and configure the polarity of said ablation probe electrodes and ablation parameters based on the polarity of drug molecules, thereby regulating the penetration speed and extent of drug molecules between said electrodes and within target tissue.

33. The ablation probe of claim 13, wherein a probe handle is provided at one end of said probe shaft; said ablation probe further comprises an outer probe shaft, which is removably fitted over the outer surface of the probe shaft of said ablation probe, connected to said probe handle, and electrically connected to said electroablation module; a first electrode is provided at the proximal end of said first light frozen zone located within the frozen zone of said outer probe shaft; a second electrode is provided at the probe tip of said second light frozen zone located at the distal end of said frozen zone.

34. The ablation probe of claim 13, wherein said ablation probe further comprises an outer sheath, which is removably fitted over the probe shaft of said ablation probe, said outer sheath is slidable axially along said probe shaft, with one end of said outer sheath connected to the probe handle of said ablation probe; said outer sheath comprises a rod-shaped portion with a first electrode positioned thereon, and a second electrode positioned at the probe tip of said ablation probe;
the outer sleeve is provided with a slider and a groove, wherein said groove is configured to position said first electrode; said slider is fixedly connected to the rod-shaped portion and is configured to drive the rod-shaped portion to slide axially along said probe shaft, thereby driving the first electrode to move correspondingly to alter the spacing and impedance between said first and second electrodes, to ensure them positioned within said light frozen zone.

35. The ablation probe of claim 34, wherein said outer sheath is a thermal insulation tube, and the lengths of said frozen zone and light frozen zone are adjustable by sliding said outer sheath, said first electrode is positioned at the distal end of said thermal insulation tube such that said first electrode is always located within the light frozen zone of said frozen zone.

36. An ablation control method of combining cryoablation and electroablation, applicable to said system of claims 1 to 12, said ablation control method are as follows:
S100: Insert said ablation probes into the target tissue, with at least two electrodes of opposite polarity positioned within said light frozen zone; or at least one electrode positioned within said light frozen zone, and at least one electrode of opposite polarity positioned within or in electrical contact with the human tissue;
S200: Control said freezing module or freezing device to issue a freezing command, to freeze the target tissue to below 0°C via said ablation probe with freezing function to form a frozen zone;
S300: Prior to, and/or concurrently with, and/or following freezing, control said electroablation module or electroablation device to issue electroablation commands to perform electroablation via said electroablation probe or electrode, firstly on said light frozen zone and subsequently on said deep-frozen zone.

37. The ablation control method of claim 36, wherein it further comprises the following steps:
S210: Said control module issues a thawing command to said freezing module, upon receiving the thawing command, the freezing module thaws the frozen zone contacted with the electrodes of said freezing or cryoelectrical probes so that the temperature around said probe shaft reaches that of said light frozen zone, thereby forming a conductive channel in said light frozen zone around said probe shaft electrodes;
S220: Once the conductive channel formed, electroablation is performed on the periphery of said light frozen zone or frozen zone;
S230: Upon completion of electroablation of said light frozen zone, proceed to ablate said deep-frozen zone.

38. The ablation control method of claim 36, wherein it further comprises the following steps:
S110: Said control module issues impedance monitoring instructions to said impedance measurement and control module, which monitors and displays information on inter-electrode impedance, and calculates and controls the ablation parameters of the electroablation;
S120: Upon receiving said information on inter-electrode impedance, said control module plans the electrode distribution, which is implemented by the operator;
S130: Upon receiving the information on inter-electrode impedance, the control module plans the electrode grouping, which is implemented by the operator.

39. The ablation control method of claim 36, wherein it further comprises the following steps:
S240: Uses a said ablation probe with freezing function to freeze the muscle or nerve tissue of the target tissue to make it a non-conductive deep-frozen zone, thereby inhibiting neural conduction and muscle contraction to achieve cryoanesthesia.
